# EUROPÄISCHE PATENTANMELDUNG

(11) **EP 3 907 250 A1**
(43) Veröffentlichungstag der Anmeldung: **10.11.2021**
(21) Anmeldenummer: 20173326.8
(22) Anmeldetag: 06.05.2020
(51) Int. Cl.: C08J 11/24, C07B 37/06, C07C 29/09, C07C 29/128, C07C 31/20, C07C 51/09, C07C 55/18, C07C 55/20, C07C 55/21, C08G 63/16, C08G 63/83, C08G 64/02, C08G 64/30

(54) **POLYESTER OR POLYCARBONAT-ARTIGE MATERIALIEN, IHRE SOLVOLYSE UND HERSTELLUNG VON FERTIGTEILEN DARAUS**

(71) Anmelder: Universitaet Konstanz, 76464 Konstanz (DE)
(72) Erfinder: Mecking, Stefan, 78464 Konstanz (DE); Häußler, Manuel, 78465 Konstanz (DE); Eck, Marcel, 78462 Konstanz (DE)
(74) Vertreter: Altmann Stößel Dick Patentanwälte PartG mbB

(57) **Zusammenfassung**

Die Erfindung betrifft gemäß eines erstes Aspekts ein Polyethylen-artiges Polymer umfassend Methylengruppen (CH₂) und mindestens eine funktionelle Gruppe ausgewählt aus O-C(=O)-Gruppe und O-C(=O)-O-Gruppe, wobei das Polyethylen-artige Polymer ein zahlenmittleres Molekulargewicht (Mₙ) im Bereich von 20.000 bis 10.000.000 g/mol aufweist.

In einem zweiten Aspekt betrifft die Erfindung ein Verfahren zur Herstellung von spezifischen Monomeren der allgemeinen Formel (II) und/oder spezifischen Oligomeren der allgemeinen Formel (III).

In einem dritten Aspekt bezieht sich die Erfindung auf eine Zusammensetzung umfassend ein Monomer der allgemeinen Formel (II) und/oder ein Oligomer der allgemeinen Formel (III), wobei die Zusammensetzung erhalten oder erhältlich ist nach dem Verfahren des zweiten Aspektes.

Ein vierter Aspekt der Erfindung bezieht sich auf die Verwendung der Zusammensetzung gemäß dem dritten Aspekt zur Herstellung von Polymeren.

Ein fünfter Aspekt der Erfindung betrifft ein Verfahren zum Herstellen eines Fertigteils, umfassend Bereitstellen eines Materials, das ein Polyethylen-artiges Polymer aufweist, und Urformen des Materials.

Ein sechster Aspekt der Erfindung bezieht sich auf ein Verfahren zum Herstellen eines Fertigteils aus einem Material, wobei das Material ein Polyethylen-artiges Polymer aufweist, wobei das Fertigteil mittels eines additiven Fertigungsverfahrens hergestellt wird.

## Beschreibung

Die Erfindung betrifft gemäß eines erstes Aspekts ein Polyethylen-artiges Polymer umfassend Methylengruppen (CH₂) und mindestens eine funktionelle Gruppe ausgewählt aus O-C(=O)-Gruppe und O-C(=O)-O-Gruppe, wobei das Polyethylen-artige Polymer ein zahlenmittleres Molekulargewicht (Mₙ) im Bereich von 20.000 bis 10.000.000 g/mol aufweist. In einem zweiten Aspekt betrifft die Erfindung ein Verfahren zur Herstellung von spezifischen Monomeren der allgemeinen Formel (II) und/oder spezifischen Oligomeren der allgemeinen Formel (III). In einem dritten Aspekt bezieht sich die Erfindung auf eine Zusammensetzung umfassend ein Monomer der allgemeinen Formel (II) und/oder ein Oligomer der allgemeinen Formel (III), wobei die Zusammensetzung erhalten oder erhältlich ist nach dem Verfahren des zweiten Aspektes. Ein vierter Aspekt der Erfindung bezieht sich auf die Verwendung der Zusammensetzung gemäß dem dritten Aspekt zur Herstellung von Polymeren. Ein fünfter Aspekt der Erfindung betrifft ein Verfahren zum Herstellen eines Fertigteils, umfassend Bereitstellen eines Materials, das ein Polyethylen-artiges Polymer aufweist, und Urformen des Materials. Ein sechster Aspekt der Erfindung bezieht sich auf ein Verfahren zum Herstellen eines Fertigteils aus einem Material, wobei das Material ein Polyethylen-artiges Polymer aufweist, wobei das Fertigteil mittels eines additiven Fertigungsverfahrens hergestellt wird.

Die Produktions- bzw. Verarbeitungsmengen der Kunststoffindustrie sind in den letzten Jahrzehnten nahezu explosiv angestiegen, wobei Kunststoffe meist aus organischen Stoffen wie Cellulose, Kohle, Erdgas und Erdöl erzeugt werden. Das Spektrum der erzeugten Polymere ist manigfaltig und umfasst diverse Duroplaste und Thermoplaste. Die Thermoplasten umfassen beispielsweise Polyolefine wie Polyethylen (PE) und Polypropylen (PP), Polyvinylchlorid (PVC), Polyester wie Polyethylenterephthalat (PET), Polystyrol und expandiertes Polystyrol (PS/PS-E). Ein Großteil der erzeugten Kunststoffe entfällt hierbei auf Polyethylen, Polypropylen und Polystyrol. Diese Gruppe besitzt somit eine hohe Relevanz, auch im Hinblick auf die Abfallwirtschaft, wenn man bedenkt, dass Polyolefine wie Polyethylen und Polypropylen den Hauptwerkstoff für Kunststoffverpackungen und -behältnisse darstellen, welche im Vergleich zu anderen Polymerprodukten eine sehr kurze Lebensdauer aufweisen.

Im Hinblick auf die Abfallmassen, die aufgrund von fehlender Abbaubarkeit ein Umweltproblem darstellen und die gleichzeitig in diesen Abfällen enthaltenen immensen Ressourcen ist ein Recycling von Kunststoffen ein zentraler Gesichtspunkt.

Insbesondere ist ein geschlossenes Recycling von Kunststoffen wünschenswert. Ein Ansatz ist die Wiederverwendung der Kunststoffe als solche, auch als "mechanisches Recycling" bezeichnet. Dies geht mit einer stark verminderten Qualität der recycelten Materialien einher. Gründe sind der Verlust des Molekulargewichts und anderer Materialeigenschaften durch die mechanischen und thermischen Behandlungsschritte, insbesondere aber die Verunreinigung mit anderen Arten und Qualitäten von Kunststoffen beim Recycling typischer Kunststoffabfallströme.

Daher ist ein chemischer Abbau in niedermolekulare Bausteine, sogenannte Oligomere und insbesondere Monomere, die als Ausgangsmaterial für eine erneute Polymerisation dienen können, um qualitativ hochwertige Materialien zu erhalten, ein wünschenswerter Ansatz (allgemein als "chemisches Recycling" bezeichnet). Chemische Recyclingverfahren sind Gegenstand der Forschung: Beim chemischen Recycling werden meist durch das Einwirken von Wärme, Katalysatoren und Lösungsmitteln die Polymere in kürzere oligomere Einheiten bis hin zu Monomeren aufgetrennt. Die dabei gewonnenen Oligomere und Monomere können dann wieder zur Kunststoffproduktion eingesetzt werden, wodurch primäre Ressourcen eingespart werden können. Verschiede Verfahren, die mittels Solvolyse und/oder Pyrolyse, mit und ohne Katalysator, arbeiten, wurden bereits beschrieben. Beispielsweise offenbart das US Patent US 6,472,557 B1 ein Recycling-Verfahren, bei welchem PET mit Zinkacetat in methanolischer Lösung unter autogenem Druck im Autoklaven erhitzt wird.

Bei Polyolefinen, insbesondere Polyethylen, geht das bisher bekannte chemische Recycling mit begrenzten Ausbeuten an Ethylenmonomer von ≤50% einher. Weiterhin wird viel Energie benötigt, da das Verfahren Temperaturen von ca. 700 ° C erfordert.

Polyethylen-artige Materialien, welche aus ungesättigten Fettsäuren erzeugt werden, wurden im Laufe der letzten Jahre bekannt. Beispielsweise beschreibt DE 10 2016 010 503 A1 ein Verfahren beruhend auf einer Kettenvervielfachung ungesättigter Fettsäuren. Langkettige aliphatische Polyester auf Basis von Pflanzenölen werden von Stempfle et al. beschrieben (F. Stempfle, B. S. Ritter, R. Mülhaupt, S. Mecking, Green Chem., 2014, 16, 2008-2014).

Ortmann et al. beschreiben die hydrolytische Abbaubarkeit von Polyethylen-artigen Polyacetalen und Polycarbonaten (P. Ortmann, I. Heckler, S. Mecking, Green Chem., 2014, 16, 1816-1827). Experimente zum hydrolytischen Abbau wurden dabei an Feststoffen in konzentrierten bzw. verdünnten, jeweils sauren oder basischen wässrigen Medien durchgeführt, wobei der Abbau der Polymere, nicht aber die Rückgewinnung der für ihre Herstellung eingesetzten Monomere, im Fokus stand.

Aufgabe der vorliegenden Erfindung war es daher, Polyethylen-artige Materialien sowie ein dazugehöriges Verfahren bereitzustellen, welches eine möglichst vollständige Rückgewinnung der Monomere aus den Polyethylen-artigen Materialien durch Solvolyse bzw. einen möglichst geschlossenen Kreislauf für die Rückgewinnung und erneute Polymerisierung solcher Monomere ermöglicht. Die Polyethylen-artigen Materialien sollen eine gute Abbaubarkeit im Solvolyse-Verfahren aufweisen.

Im Folgenden werden die Begriffe "haben", "aufweisen", "umfassen" oder "einschließen" oder beliebige grammatikalische Abweichungen davon in nicht-ausschließlicher Weise verwendet. Dementsprechend können sich diese Begriffe sowohl auf Situationen beziehen, in welchen, neben den durch diese Begriffe eingeführten Merkmalen, keine weiteren Merkmale vorhanden sind, oder auf Situationen, in welchen ein oder mehrere weitere Merkmale vorhanden sind. Beispielsweise kann sich der Ausdruck "A hat B", "A weist B auf", "A umfasst B" oder "A schließt B ein" sowohl auf die Situation beziehen, in welcher, abgesehen von B, kein weiteres Element in A vorhanden ist (d.h. auf eine Situation, in welcher A ausschließlich aus B besteht), als auch auf die Situation, in welcher, zusätzlich zu B, ein oder mehrere weitere Elemente in A vorhanden sind, beispielsweise Element C, Elemente C und D oder sogar weitere Elemente.

Weiterhin wird darauf hingewiesen, dass die Begriffe "mindestens ein" und "ein oder mehrere" sowie grammatikalische Abwandlungen dieser Begriffe, wenn diese in Zusammenhang mit einem oder mehreren Elementen oder Merkmalen verwendet werden und ausdrücken sollen, dass das Element oder Merkmal einfach oder mehrfach vorgesehen sein kann, in der Regel lediglich einmalig verwendet werden, beispielsweise bei der erstmaligen Einführung des Merkmals oder Elementes. Bei einer nachfolgenden erneuten Erwähnung des Merkmals oder Elementes wird der entsprechende Begriff "mindestens ein" oder "ein oder mehrere" in der Regel nicht mehr verwendet, ohne dass hierdurch die Möglichkeit eingeschränkt wird, dass das Merkmal oder Element einfach oder mehrfach vorgesehen sein kann.

Weiterhin werden im Folgenden die Begriffe "vorzugsweise", "insbesondere", "beispielsweise" oder ähnliche Begriffe in Verbindung mit optionalen Merkmalen verwendet, ohne dass alternative Ausführungsformen hierdurch beschränkt werden. So sind Merkmale, welche durch diese Begriffe eingeleitet werden, optionale Merkmale, und es ist nicht beabsichtigt, durch diese Merkmale den Schutzumfang der Ansprüche und insbesondere der unabhängigen Ansprüche einzuschränken. So kann die Erfindung, wie der Fachmann erkennen wird, auch unter Verwendung anderer Ausgestaltungen durchgeführt werden. In ähnlicher Weise werden Merkmale, welche durch "in einer Ausführungsform der Erfindung" oder durch "in einem Ausführungsbeispiel der Erfindung" eingeleitet werden, als optionale Merkmale verstanden, ohne dass hierdurch alternative Ausgestaltungen oder der Schutzumfang der unabhängigen Ansprüche eingeschränkt werden soll. Weiterhin sollen durch diese einleitenden Ausdrücke sämtliche Möglichkeiten unangetastet bleiben, die hierdurch eingeleiteten Merkmale mit anderen Merkmalen zu kombinieren, seien es optionale oder nicht-optionale Merkmale.

### 1. Aspekt - Polyethylen-artiges Polymer

Gemäß eines ersten Aspekts betrifft die Erfindung ein Polyethylen-artiges Polymer umfassend Methylengruppen (CH₂) und mindestens eine funktionelle Gruppe ausgewählt aus O-C(=O)-Gruppe und O-C(=O)-O-Gruppe, wobei das Polyethylen-artige Polymer ein zahlenmittleres Molekulargewicht (Mₙ) im Bereich von 20.000 bis 10.000.000 g/mol aufweist.

Das zahlenmittlere Molekulargewicht Mₙ wird bestimmt mittels Gelpermeationschromatographie (GPC) gegen Polystyrol-Standards im Bereich von 400 - 5.000.000 g/mol (universelle Kalibrierung).

Bevorzugt bestehen 90 bis 99,5 Mol-%, weiter bevorzugt 92 bis 98 Mol-%, des Polyethylen-artigen Polymers aus Methyleneinheiten (CH₂).

Gemäß einer bevorzugten Ausführungsform des Polyethylen-artigen Polymers weist dieses eine Wiederholungseinheit der allgemeinen Formel (I)

[-X-(CH₂)ₐ-] (I)

auf, wobei
X ausgewählt ist aus C(=O)-O-Gruppe bzw. -O-C(=O)-Gruppe und-O-C(=O)-O-Gruppe;
a eine ganze Zahl aus dem Bereich von 8 bis 200 ist. In einer bevorzugten Ausführungsform ist die allgemeine Formel (I) wie folgt: [-X-(CH₂)ₐ-]ₙ, wobei "X" und "a" die angegebene Bedeutung haben.

Der Index "a" kann bei jeder der n Wiederholungseinheiten gleich oder verschieden sein, d.h. bei n Wiederholungseinheiten gibt es maximal n verschiedene Werte für den Index "a". "X" kann bei jeder der n Wiederholungseinheiten gleich oder verschieden sein, d.h. jede der n X-Gruppen ist unabhängig ausgewählt aus C(=O)-O-Gruppe bzw. -O-C(=O)-Gruppe und-O-C(=O)-O-Gruppe.

Der Index "n", der bei der Wiederholungseinheit der allgemeinen Formel (I) angegeben ist, steht bevorzugt für eine ganze Zahl aus dem Bereich von 8 bis 60.000 (90 bis 99.5 mol% Methylengruppen im Polymer), weiter bevorzugt aus dem Bereich von 28 bis 47.000 (92 bis 98 mol% Methylengruppen im Polymer).

Gemäß einer weiter bevorzugten Ausführungsform des Polyethylen-artigen Polymers weist dieses eine Wiederholungseinheit der allgemeinen Formel I (la)

[X¹-(CH₂)ₐ-]ₙ (la)

auf, wobei
- X¹: ausgewählt ist aus C(=O)-O-Gruppe und -O-C(=O)-Gruppe;
- a: eine ganze Zahl aus dem Bereich von 8 bis 200 ist.

Polyethylen-artige Polymere aufweisend eine Wiederholungseinheit der allgemeinen Formel I (la) werden auch als "Polyester" bezeichnet. Der Index "a" kann bei jeder der n Wiederholungseinheiten der allgemeinen Formel (la) gleich oder verschieden sein, d.h. bei n Wiederholungseinheiten gibt es maximal n verschiedene Werte für den Index "a". "X¹" kann bei jeder der n Wiederholungseinheiten gleich oder verschieden sein, d.h. jede der n X¹-Gruppen ist unabhängig ausgewählt aus C(=O)-O-Gruppe und -O-C(=O)-Gruppe.

Der Index "n", der bei der Wiederholungseinheit der allgemeinen Formel (la) angegeben ist, steht für eine ganze Zahl aus dem Bereich von 8 bis 60.000, bevorzugt aus dem Bereich von 8 bis 58.562 - dies entspricht bei einem Mₙ im Bereich von 20.000 bis 10.000.000 g/mol einem Prozentsatz von 90 - 99.5 mol% Methylengruppen im Polymer, entsprechend 8 - 200 Methylengruppen pro funktioneller Gruppe in (la). In einer weiter bevorzugten Ausführungsform steht n für eine ganze Zahl aus dem Bereich von 18 bis 60.000, bevorzugt aus dem Bereich von 18 bis 58.562 - dies entspricht bei einem Mₙ im Bereich von 50.000 bis 10.000.000 g/mol einem Prozentsatz von 90 - 99.5 mol% Methylengruppen im Polymer.

Weiter bevorzugt steht n bei (la) für eine ganze Zahl aus dem Bereich von 28 bis 47.000, weiter bevorzugt aus dem Bereich von 28 bis 46.984 - dies entspricht bei einem Mₙ im Bereich von 20.000 bis 10.000.000 g/mol einem Prozentsatz von 92 - 98 mol% Methylengruppen im Polymer, entsprechend 12 - 49 Methylengruppen pro funktioneller Gruppe in (la). In einer weiter bevorzugten Ausführungsform steht n für eine ganze Zahl aus dem Bereich von 70 bis 47.000, weiter bevorzugt aus dem Bereich von 70 bis 46.984 - dies entspricht bei einem Mₙ im Bereich von 50.000 bis 10.000.000 g/mol einem Prozentsatz von 92 - 98 mol% Methylengruppen im Polymer. Für Polyethylen-artige Polymere aufweisend eine Wiederholungseinheit der allgemeinen Formel (la) ist es bevorzugt, dass das zahlenmittlere Molekulargewicht im Bereich von 50.000 bis 10.000.000 g/mol liegt.

Gemäß einer alternativen, weiter bevorzugten Ausführungsform des Polyethylen-artigen Polymers weist dieses eine Wiederholungseinheit der allgemeinen Formel (Ib)

[-O-C(=O)-O-(CH₂)ₐ-]ₙ (Ib)

auf, wobei
- a: eine ganze Zahl aus dem Bereich von 8 bis 200 ist.
Polyethylen-artige Polymere aufweisend eine Wiederholungseinheit der allgemeinen Formel I (Ib) werden auch als "Polycarbonate" bezeichnet. Der Index "a" kann bei jeder der n Wiederholungseinheiten der allgemeinen Formel (Ib) gleich oder verschieden sein, d.h. bei n Wiederholungseinheiten gibt es maximal n verschiedene Werte für den Index "a". Der Index "n", der bei der Wiederholungseinheit der allgemeinen Formel (Ib) angegeben ist, steht für eine ganze Zahl aus dem Bereich von 8 bis 60.000, bevorzugt aus dem Bereich von 8 bis 53.546 - dies entspricht bei einem Mₙ im Bereich von 20.000 bis 10.000.000 g/mol einem Prozentsatz von 90 - 99.5 mol% Methylengruppen im Polymer, entsprechend 8 - 200 Methylengruppen pro funktioneller Gruppe in (Ib). Weiter bevorzugt steht n bei (Ib) für eine ganze Zahl aus dem Bereich von 28 bis 47.000, weiter bevorzugt aus dem Bereich von 28 bis 43.698 - dies entspricht bei einem Mₙ im Bereich von 20.000 bis 10.000.000 g/mol einem Prozentsatz von 92 - 98 mol% Methylengruppen, entsprechend 12 - 49 Methylengruppen pro funktioneller Gruppe in (Ib). Für Polyethylen-artige Polymere aufweisend eine Wiederholungseinheit der allgemeinen Formel (Ib) ist es bevorzugt, dass das zahlenmittlere Molekulargewicht im Bereich von 20.000 bis 10.000.000 g/mol liegt.

### 2. Aspekt - Solvolyse-Verfahren

Gemäß eines zweiten Aspektes betrifft die vorliegende Erfindung ein Verfahren zur Herstellung von Monomeren der allgemeinen Formel (II) und/oder Oligomeren der allgemeinen Formel (III)

Y¹-(CH₂)_{c}-Y² (II)

wobei Y¹ und Y² jeweils unabhängig voneinander ausgewählt sind aus R-O-C(=O)-Gruppe bzw. -C(=O)-O-R-Gruppe und OH-Gruppe, wobei R ein Wasserstoffatom oder eine C1-C10-Alkylgruppe, bevorzugt ein Wasserstoffatom oder eine C1-C5-Alkylgruppe, weiter bevorzugt ein Wasserstoffatom oder eine C1- oder C2-Alkylgruppe, ist; wobei die Reste Y¹, Y² bevorzugt jeweils gleich sind, und der Index c eine ganze Zahl aus dem Bereich von 8 bis 200 ist,

Y¹-[(CH₂)ₐ-X-]ₘ-Y² (III)

wobei Y¹ und Y² jeweils unabhängig voneinander ausgewählt sind aus R-O-C(=O)-O- Gruppe bzw. -O-C(=O)-O-R-Gruppe, R-O-C(=O)-Gruppe bzw. -C(=O)-O-R-Gruppe und OH-Gruppe, wobei R ein Wasserstoffatom oder eine C1-C10-Alkylgruppe, bevorzugt ein Wasserstoffatom oder eine C1-C5-Alkylgruppe, weiter bevorzugt ein Wasserstoffatom oder eine C1- oder C2-Alkylgruppe, ist; X aus C(=O)-O-Gruppe bzw. -O-C(=O)-Gruppe und -O-C(=O)-O-Gruppe ausgewählt ist, wobei X in der letzten Wiederholungseinheit von Formel (III), d.h. beim Übergang zu Y², nicht vorhanden ist; a eine ganze Zahl aus dem Bereich von 8 bis 200 ist; und der Index m eine ganze Zahl aus dem Bereich von 2 bis 40 ist;
wobei das Verfahren umfasst:
i) Erzeugen einer alkoholischen und/oder wässrigen Mischung eines Polyethylen-artigen Polymers umfassend Methylengruppen und mindestens eine funktionelle Gruppe ausgewählt aus O-C(=O)-Gruppe und O-C(=O)-O-Gruppe;
ii) Erhitzen der gemäß i) bereitgestellten Mischung, bevorzugt unter autogenem Druck, auf eine Temperatur im Bereich von 20 bis 400 °C, bevorzugt 60 bis 250 °C, weiter bevorzugt 80 bis 180 °C;
iii) Abtrennen der Monomere der allgemeinen Formel (II) und/oder Oligomere der allgemeinen Formel (III) aus bzw. von der gemäß ii) erhaltenen alkoholischen und/oder wässrigen Mischung.

Der Index "a" kann bei jeder der m Wiederholungseinheiten der allgemeinen Formel (III) gleich oder verschieden sein, d.h. bei m Wiederholungseinheiten gibt es maximal m verschiedene Werte für den Index "a". "X" kann bei jeder der n Wiederholungseinheiten gleich oder verschieden sein, d.h. jede der m X-Gruppen ist unabhängig ausgewählt aus C(=O)-O-Gruppe bzw. -O-C(=O)-Gruppe und-O-C(=O)-O-Gruppe.

X ist in den Oligomeren der allgemeinen Formel (III) nicht bei der letzten Wiederholungseinheit vorhanden. Dies bedeutet exemplarisch für m = 2 den folgenden Aufbau eines Oligomers der allgemeinen Formel (III):

Y¹-[(CH₂)ₐ-X-]-[(CH₂)ₐ-]-Y²

Der Aufbau für Oligomere der allgemeinen Formel (III) mit m im Bereich von 3 bis 40 ist analog.

Das voranstehend beschriebene Verfahren wird auch als "Solvolyse-Verfahren" bezeichnet. Wie eingangs beschrieben weisen viele bekannte Recycling-Verfahren diverse Nachteile auf: Bei einem chemischen Recycling ist oft nachteilig, dass die Rückgewinnung der eingesetzten Monomere aus dem Plastik nur in schlechten Ausbeuten gelingt bzw. sich durch Nebenreaktionen etc. diverse andere Abbauprodukte ergeben, die dann wieder bei einer erneuten Polymerisation stören. Weiterhin benötigt beispielsweise die Rückgewinnung von Ethen aus Polyethylen einen hohen energetischen Aufwand, da Temperaturen von ca. 700 °C erforderlich sind.

Für das erfindungsgemäße Solvolyse-Verfahren konnte überraschenderweise gezeigt werden, dass eine Rückgewinnung von Monomeren und/oder Oligomeren aus dem Polyethylen-artigen Polymer bei deutlich milderen Bedingungen, u.a. Temperaturen deutlich unterhalb von 700 °C, möglich ist und dass weiterhin nur ein geringer energetischer Aufwand erforderlich ist. Hinzu kommt, dass die Monomere und/oder Oligomere in hohen Selektivitäten und in hoher Qualität erhalten werden. Selbst Farbstoffe und weitere Bestandteile, die dem Polyethylen-artigen Polymer zugesetzt sind, können unproblematisch abgetrennt werden. Abgetrennt werden können weiterhin quantitativ andere Polymere wie zum Beispiel Polyolefine, insbesondere Polypropylen bzw. Polyethylen. Diese anderen Polymere, insbesondere Polyolefine, liegen nach erfolgter Solvolysestufe (ii) neben den Monomere der allgemeinen Formel (II) und/oder Oligomere der allgemeinen Formel (III) unverändert in polymerer Form vor. Die Monomere der allgemeinen Formel (II) und/oder Oligomere der allgemeinen Formel (III) können von diesen unverändert in polymerer Form vorliegenden Stoffen auf einfachem Wege abgetrennt werden. Das erfindungsgemäße Solvolyse-Verfahren ermöglicht es, ein mehrzyklisches Recycling durchzuführen, d.h. es sind mehre Durchgänge von Polymerisation von Monomeren, Solvolyse und erneuter Polymerisation der erhaltenen Monomere und/oder Oligomere etc. möglich. In anderen Worten ist ein so genanntes "Closed-Loop Recycling" möglich.

Der Begriff der alkoholischen und/oder wässrigen Mischung gemäß (i) umfasst Lösungen und heterogene Gemische von Alkohol und oder Wasser mit dem Polyethylen-artigen Polymer. Analog gilt für die gemäß ii) erhaltenen alkoholischen und/oder wässrigen Mischungen, dass sie Lösungen und heterogene Gemische von Alkohol und oder Wasser mit den Monomeren der allgemeinen Formel (II) und/oder Oligomeren der allgemeinen Formel (III) umfasst.

Das für das Verfahren eingesetzte Polyethylen-artige Polymer weist ein zahlenmittleres Molekulargewicht (Mₙ) im Bereich von 5.000 bis 10.000.000 g/mol, bevorzugt im Bereich von 10.000 bis 10.000.000 g/mol auf. In bevorzugten Ausführungsformen weist es ein zahlenmittleres Molekulargewicht (Mₙ) im Bereich von 20.000 bis 10.000.000 g/mol oder im Bereich von 50.000 bis 10.000.000 g/mol, auf.

Die Bestimmung von Mₙ erfolgt auch im Rahmen des zweiten Aspektes der Erfindung mittels Gelpermeationschromatographie (GPC) gegen Polystyrol-Standards im Bereich von 400 - 5.000.000 g/mol (universelle Kalibrierung).

Bevorzugt umfasst das Verfahren weiterhin:
iv) optionale Aufreinigung der gemäß iii) erhaltenen Monomere der allgemeinen Formel (II) und/oder Oligomere der allgemeinen Formel (III);
v) Repolymerisation der gemäß iii) oder iv) erhaltenen Monomere der allgemeinen Formel (II) und/oder Oligomere der allgemeinen Formel (III) zu einem Polymer, bevorzugt zu einem Polyethylen-artigen Polymer.

Ein solches Solvolyse-Verfahren umfassend Schritt (v) stellt ein geschlossenes Recycling dar, wobei nach Schritt (v) ein Polymer vorliegt, welches einem direkt aus den passenden Monomeren erzeugten Polymer gleichwertig ist, d.h. es gibt keine Verschlechterung der mechanischen und chemischen Eigenschaften des Materials.

In einer bevorzugten Ausführungsform des Verfahrens zur Herstellung von Monomeren der allgemeinen Formel (II) und/oder Oligomeren der allgemeinen Formel (III) weist das Polyethylen-artige Polymer umfassend Methylengruppen und mindestens eine funktionelle Gruppe ausgewählt aus O-C(=O)-Gruppe und O-C(=O)-O-Gruppe, eine Wiederholungseinheit der allgemeinen Formel (I) auf

[-X-(CH₂)ₐ-] (I),

wobei
X ausgewählt ist aus C(=O)-O-Gruppe bzw. -O-C(=O)-Gruppe und-O-C(=O)-O-Gruppe;
a eine ganze Zahl aus dem Bereich von 8 bis 200 ist. In einer bevorzugten Ausführungsform ist die allgemeine Formel (I) wie folgt: [-X-(CH₂)ₐ-]ₙ, wobei "X" und "a" die angegebene Bedeutung haben.

Der Index "a" kann bei jeder der n Wiederholungseinheiten der allgemeinen Formel (I) gleich oder verschieden sein, d.h. bei n Wiederholungseinheiten gibt es maximal n verschiedene Werte für den Index "a". X" kann bei jeder der n Wiederholungseinheiten gleich oder verschieden sein, d.h. jede der n X-Gruppen ist unabhängig ausgewählt aus C(=O)-O-Gruppe bzw. -O-C(=O)-Gruppe und-O-C(=O)-O-Gruppe. Der Index "n", der bei der Wiederholungseinheit der allgemeinen Formel (I) angegeben ist, steht für eine ganze Zahl aus dem Bereich von 2 bis 60.000 (90 bis 99.5 mol% Methylengruppen im Polymer bei einem Mₙ im Bereich von 5.000 bis 10.000.000 g/mol). Weiter bevorzugt steht n für eine ganze Zahl aus dem Bereich von 4 bis 60.000 (90 bis 99.5 mol% Methylengruppen im Polymer bei einem Mₙ im Bereich von 10.000 bis 10.000.000 g/mol), weiter bevorzugt steht n für eine ganze Zahl aus dem Bereich von 8 bis 60.000 (90 bis 99.5 mol% Methylengruppen im Polymer bei einem Mₙ im Bereich von 20.000 bis 10.000.000 g/mol), weiter bevorzugt steht n für eine ganze Zahl aus dem Bereich von 18 bis 60.000 (90 bis 99.5 mol% Methylengruppen im Polymer bei einem Mₙ im Bereich von 50.000 bis 10.000.000 g/mol).

Bevorzugt steht n für eine ganze Zahl aus dem Bereich von 8 bis 47.000 (92 bis 98 mol% Methylengruppen im Polymer bei einem Mₙ im Bereich von 5.000 bis 10.000.000 g/mol), weiter bevorzugt aus dem Bereich von 14 bis 47.000 (92 bis 98 mol% Methylengruppen im Polymer bei einem Mₙ im Bereich von 10.000 bis 10.000.000 g/mol), weiter bevorzugt aus dem Bereich von 28 bis 47.000 (92 bis 98 mol% Methylengruppen im Polymer bei einem Mₙ im Bereich von 20.000 bis 10.000.000 g/mol), weiter bevorzugt aus dem Bereich von 68 bis 47.000 (92 bis 98 mol% Methylengruppen im Polymer bei einem Mₙ im Bereich von 50.000 bis 10.000.000 g/mol).

Bevorzugt im Rahmen des Verfahrens zur Herstellung von Monomeren der allgemeinen Formel (II) und/oder Oligomeren der allgemeinen Formel (III) wie voranstehend beschrieben ist es, dass der Alkohol ausgewählt ist aus der Gruppe der monofunktionellen oder polyfunktionellen C1-C10-Alkohole und deren Mischungen, wobei der Alkohol bevorzugt einen oder mehrere Alkohole der Formel R-OH umfasst, wobei der Rest R eine C1-C10-Alkylgruppe, bevorzugt eine C1-C5-Alkylgruppe, weiter bevorzugt eine C1- oder C2-Alkylgruppe, ist; wobei der Alkohol weiter bevorzugt Methanol umfasst.

In einer bevorzugten Ausführungsform des Verfahrens zur Herstellung von Monomeren der allgemeinen Formel (II) und/oder Oligomeren der allgemeinen Formel (III) wie voranstehend beschrieben wird für die Erzeugung der Mischung gemäß (i) Wasser verwendet.

In einer weiteren bevorzugten Ausführungsform des Verfahrens zur Herstellung von Monomeren der allgemeinen Formel (II) und/oder Oligomeren der allgemeinen Formel (III) wird eine alkoholische Mischung verwendet, wobei die alkoholische Mischung des Polyethylen-artigen Polymers gemäß i) einen Wassergehalt im Bereich von 0 bis 5 Gewichts-%, bevorzugt im Bereich von 0 bis 1 Gewichts-%, weiter bevorzugt im Bereich von 0 bis 0,55 Gewichts-%, weiter bevorzugt von im Bereich von 0 bis 0,1 Gewichts-%, aufweist, bezogen auf das Gesamtgewicht der alkoholischen Mischung.

In einer bevorzugten Ausführungsform des Verfahrens zur Herstellung von Monomeren der allgemeinen Formel (II) und/oder Oligomeren der allgemeinen Formel (III) wie voranstehend beschrieben umfasst die alkoholische und/oder wässrige Mischung des Polyethylen-artigen Polymers gemäß i) im Bereich von 0 bis 20 Gewichts-%, bevorzugt im Bereich von 0 bis 15 Gewichts-%, weiter bevorzugt im Bereich von 0 bis 10 Gewichts-%, jeweils bezogen auf das Gewicht des eingesetzten Polyethylen-artigen Polymers, eines Katalysators, bevorzugt eines basischen Katalysators, wobei der basische Katalysator bevorzugt ausgewählt ist aus der Gruppe der Alkalihydroxide, weiter bevorzugt mindestens KOH umfasst.

In einer bevorzugten Ausführungsform des Verfahrens zur Herstellung von Monomeren der allgemeinen Formel (II) und/oder Oligomeren der allgemeinen Formel (III) wie voranstehend beschrieben erfolgt Schritt ii) bei einem Druck im Bereich von 0 bis 500 bar, bevorzugt 0,1 bis 200 bar, weiter bevorzugt 0,5 bis 100 bar, weiter bevorzugt 1 bis 20 bar. Bevorzugt erfolgt Schritt ii) in einem, bevorzugt geschlossenen, System, weiter bevorzugt in einem Autoklaven. Bevorzugt wird Schritt ii) für eine Zeitspanne im Bereich von 10 Sekunden bis 7 Tagen, bevorzugt 5 Minuten bis 24 Stunden, bevorzugt von 30 Minuten bis 2 Stunden, durchgeführt.

In einer bevorzugten Ausführungsform des Verfahrens zur Herstellung von Monomeren der allgemeinen Formel (II) und/oder Oligomeren der allgemeinen Formel (III) wie voranstehend beschrieben wird nach ii) und vor iii) die gemäß ii) erhaltene alkoholische und/oder wässrige Mischung umfassend Monomere der allgemeinen Formel (II) abgekühlt, bevorzugt auf eine Temperatur im Bereich von -100 bis + 200 °C, bevorzugt -60 bis +60 °C, weiter bevorzugt -30 bis +30 °C, und/oder der Druck auf einen Wert im Bereich von 0,8 bis 1,2 bar reduziert wird.

Das Abtrennen der Monomere der allgemeinen Formel (II) und/oder Oligomeren der allgemeinen Formel (III) gemäß iii) erfolgt bevorzugt mittels einer oder mehrerer Verfahren aus der Gruppe bestehend aus Fällung, bevorzugt mittels Fällungsreagentien, Filtration, Kristallisation, Zentrifugation, Destillation, Eindampfen und Chromatographie.

In einer bevorzugten Ausführungsform des Verfahrens zur Herstellung von Monomeren der allgemeinen Formel (II) und/oder Oligomeren der allgemeinen Formel (III) wie voranstehend beschrieben weist das Polyethylen-artige Polymer eine Wiederholungseinheit der allgemeinen Formel (la)

[X¹-(CH₂)ₐ-]ₙ. (la)

auf, wobei
- X¹: ausgewählt ist aus C(=O)-O-Gruppe und -O-C(=O)-Gruppe;
- a: eine ganze Zahl aus dem Bereich von 8 bis 200 ist.

Polyethylen-artige Polymere, welche eine Wiederholungseinheit der allgemeinen Formel (la) aufweisen, werden auch als "Polyester" bezeichnet. Der Index "a" kann bei jeder der n Wiederholungseinheiten der allgemeinen Formel (la) gleich oder verschieden sein, d.h. bei n Wiederholungseinheiten gibt es maximal n verschiedene Werte für den Index "a". "X¹" kann bei jeder der n Wiederholungseinheiten gleich oder verschieden sein, d.h. jede der n X¹-Gruppen ist unabhängig ausgewählt aus C(=O)-O-Gruppe und -O-C(=O)-Gruppe. Der Index "n", der bei der Wiederholungseinheit der allgemeinen Formel (la) angegeben ist, steht für eine ganze Zahl aus dem Bereich von 8 bis 60.000, weiter bevorzugt aus dem Bereich von 2 bis 58.562 - dies entspricht bei einem Mₙ im Bereich von 5.000 bis 10.000.000 g/mol einem Prozentsatz von 90 - 99.5 mol% Methylengruppen im Polymer. Weiter bevorzugt ist n bei (la) eine ganze Zahl aus dem Bereich von 4 bis 60.000, weiter bevorzugt aus dem Bereich von 4 bis 58.562 - dies entspricht bei einem Mₙ im Bereich von 10.000 bis 10.000.000 g/mol einem Prozentsatz von 90 - 99.5 mol% Methylengruppen im Polymer. Weiter bevorzugt steht n bei (Ia) für eine ganze Zahl aus dem Bereich von 8 bis 60.000, weiter bevorzugt aus dem Bereich von 8 bis 58.562 - dies entspricht bei einem Mₙ im Bereich von 20.000 bis 10.000.000 g/mol einem Prozentsatz von 90 - 99.5 mol% Methylengruppen im Polymer, entsprechend 8 - 200 Methylengruppen pro funktioneller Gruppe in (la). In einer weiter bevorzugten Ausführungsform steht n bei (la) für eine ganze Zahl aus dem Bereich von 18 bis 60.000, weiter bevorzugt aus dem Bereich von 18 bis 58.562 - dies entspricht bei einem Mₙ im Bereich von 50.000 bis 10.000.000 g/mol einem Prozentsatz von 90 - 99.5 mol% Methylengruppen im Polymer.

Bevorzugt steht n bei (la) für eine ganze Zahl aus dem Bereich von 8 bis 47.000, weiter bevorzugt aus dem Bereich von 8 bis 46.984 - dies entspricht bei einem Mₙ im Bereich von 5.000 bis 10.000.000 g/mol einem Prozentsatz von 92 - 98 mol% Methylengruppen im Polymer. Weiter bevorzugt steht n bei (la) für eine ganze Zahl aus dem Bereich von 14 bis 47.000, weiter bevorzugt aus dem Bereich von 14 bis 46.984 - dies entspricht bei einem Mₙ im Bereich von 10.000 bis 10.000.000 g/mol einem Prozentsatz von 92 - 98 mol% Methylengruppen im Polymer. In einer weiter bevorzugten Ausführungsform steht n bei (la) für eine ganze Zahl aus dem Bereich von 28 bis 47.000, weiter bevorzugt aus dem Bereich von 28 bis 46.984 - dies entspricht bei einem Mₙ im Bereich von 20.000 bis 10.000.000 g/mol einem Prozentsatz von 92 - 98 mol% Methylengruppen im Polymer, entsprechend 12 - 49 Methylengruppen pro funktioneller Gruppe in (la). In einer weiter bevorzugten Ausführungsform steht n bei (la) für eine ganze Zahl aus dem Bereich von 70 bis 47.000, weiter bevorzugt aus dem Bereich von 70 bis 46.984 - dies entspricht bei einem Mₙ im Bereich von 50.000 bis 10.000.000 g/mol einem Prozentsatz von 92 - 98 mol% Methylengruppen im Polymer.

In einer besonders bevorzugten Ausführungsform des Verfahrens zur Herstellung von Monomeren der allgemeinen Formel (II) und/oder Oligomeren der allgemeinen Formel (III) wie voranstehend beschrieben weist das Polyethylen-artige Polymer eine Wiederholungseinheit der allgemeinen Formel (Ib)

[-O-C(=O)-O-(CH₂)ₐ-]ₙ (Ib)

auf, wobei
- a: eine ganze Zahl aus dem Bereich von 8 bis 200 ist.
Polyethylen-artige Polymere, welche eine Wiederholungseinheit der allgemeinen Formel (Ib) aufweisen, werden auch als "Polycarbonate" bezeichnet. Der Index "a" kann bei jeder der n Wiederholungseinheiten der allgemeinen Formel (Ib) gleich oder verschieden sein, d.h. bei n Wiederholungseinheiten gibt es maximal n verschiedene Werte für den Index "a". Der Index "n", der bei der Wiederholungseinheit der allgemeinen Formel (Ib) angegeben ist, steht für eine ganze Zahl aus dem Bereich von 2 bis 60.000, weiter bevorzugt aus dem Bereich von 2 bis 56.000, weiter bevorzugt aus dem Bereich von 2 bis 53.546 - dies entspricht bei einem Mₙ im Bereich von 5.000 bis 10.000.000 g/mol einem Prozentsatz von 90 - 99.5 mol% Methylengruppen im Polymer. Weiter bevorzugt steht n bei (Ib) für eine ganze Zahl aus dem Bereich von 4 bis 60.000, weiter bevorzugt aus dem Bereich von 4 bis 53.546 - dies entspricht bei einem Mₙ im Bereich von 10.000 bis 10.000.000 g/mol einem Prozentsatz von 90 - 99.5 mol% Methylengruppen im Polymer. Weiter bevorzugt steht n bei (Ib) für eine ganze Zahl aus dem Bereich von 8 bis 60.000, weiter bevorzugt aus dem Bereich von 8 bis 53.546 - dies entspricht bei einem Mₙ im Bereich von 20.000 bis 10.000.000 g/mol einem Prozentsatz von 90 - 99.5 mol% Methylengruppen im Polymer, entsprechend8 - 200 Methylengruppen pro funktioneller Gruppe in (Ib). Weiter bevorzugt steht n bei (Ib) für eine ganze Zahl aus dem Bereich von 18 bis 60.000, weiter bevorzugt aus dem Bereich von 18 bis 53.546 - dies entspricht bei einem Mₙ im Bereich von 50.000 bis 10.000.000 g/mol einem Prozentsatz von 90 - 99.5 mol% Methylengruppen im Polymer.

Bevorzugt steht n bei (Ib) für eine ganze Zahl aus dem Bereich von 8 bis 47.000, weiter bevorzugt aus dem Bereich von 8 bis 43.698 - dies entspricht bei einem Mₙ im Bereich von 5.000 bis 10.000.000 g/mol einem Prozentsatz von 92 - 98 mol% Methylengruppen im Polymer. Weiter bevorzugt steht n bei (Ib) für eine ganze Zahl aus dem Bereich von 14 bis 47.000, weiter bevorzugt aus dem Bereich von 14 bis 43.698 - dies entspricht bei einem Mₙ im Bereich von 10.000 bis 10.000.000 g/mol einem Prozentsatz von 92 - 98 mol% Methylengruppen im Polymer. Weiter bevorzugt steht n bei (Ib) für eine ganze Zahl aus dem Bereich von 28 bis 47.000, weiter bevorzugt aus dem Bereich von 28 bis 43.698 - dies entspricht bei einem Mₙ im Bereich von 20.000 bis 10.000.000 g/mol einem Prozentsatz von 92 - 98 mol% Methylengruppen im Polymer, entsprechend 12 - 49 Methylengruppen pro funktioneller Gruppe in (Ib). Weiter bevorzugt steht n bei (Ib) für eine ganze Zahl aus dem Bereich von 68 bis 47.000, weiter bevorzugt dem Bereich von 68 bis 43.698 - dies entspricht bei einem Mₙ im Bereich von 50.000 bis 10.000.000 g/mol einem Prozentsatz von 92 - 98 mol% Methylengruppen im Polymer.

Bevorzugt umfasst die in ii) erhaltene alkoholische und/oder wässrige Mischung umfassend Monomere der allgemeinen Formel (II) und/oder Oligomere der allgemeinen Formel (III) weiterhin Monomere der allgemeinen Formel (IV)

R¹-O-C(=O)-O-R² (IV)

, wobei
R¹, R² jeweils eine C1-C10-Alkylgruppe, bevorzugt eine C1-C5-Alkylgruppe, weiter bevorzugt eine C1- oder C2-Alkylgruppe, sind; wobei die Monomere der allgemeinen Formel (IV) bevorzugt in iii) zusammen mit den Monomeren der allgemeinen Formel (II) und/oder Oligomeren der allgemeinen Formel (III) aus der gemäß ii) erhaltenen alkoholischen und/oder wässrigen Mischung abgetrennt werden.

Bevorzugt werden die Monomere der allgemeinen Formel (II) und/oder die Oligomere der allgemeinen Formel (III) in einer Ausbeute von ≥ 90 Mol-%, bevorzugt von ≥ 92 Mol-%, weiter bevorzugt von ≥ 94 Mol-%, weiter bevorzugt von ≥ 96 Mol-%, bezogen auf das eingesetzte Polyethylen-artige Polymer, erhalten.

### 3. Aspekt - Zusammensetzung

Ein dritter Aspekt der Erfindung betrifft eine Zusammensetzung umfassend ein Monomer der allgemeinen Formel (II) und/oder ein Oligomer der allgemeinen Formel (III)

Y¹-(CH₂)_{c}-Y² (II)

wobei Y¹ und Y² jeweils unabhängig voneinander ausgewählt sind aus R-O-C(=O)-Gruppe bzw. -C(=O)-O-R-Gruppe und OH-Gruppe, wobei R ein Wasserstoffatom oder eine C1-C10-Alkylgruppe, bevorzugt ein Wasserstoffatom oder eine C1-C5-Alkylgruppe, weiter bevorzugt ein Wasserstoffatom oder eine C1- oder C2-Alkylgruppe, ist; und der Index c eine ganze Zahl aus dem Bereich von 8 bis 200 ist,

Y¹-[(CH₂)ₐ-X-]ₘ-Y² (III)

, wobei Y¹ und Y² jeweils unabhängig voneinander ausgewählt sind aus R-O-C(=O)-O-Gruppe bzw. -O-C(=O)-O-R-Gruppe, R-O-C(=O)-Gruppe bzw. -C(=O)-O-R-Gruppe und OH-Gruppe, wobei R ein Wasserstoffatom oder eine C1-C10-Alkylgruppe, bevorzugt ein Wasserstoffatom oder eine C1-C5-Alkylgruppe, weiter bevorzugt ein Wasserstoffatom oder eine C1- oder C2-Alkylgruppe, ist; X aus C(=O)-O-Gruppe bzw. -O-C(=O)-Gruppe und-O-C(=O)-O-Gruppe ausgewählt ist, wobei X in der letzten Wiederholungseinheit von Formel (III), d.h. beim Übergang zu Y², nicht vorhanden ist; a eine ganze Zahl aus dem Bereich von 8 bis 200 ist; und der Index m eine ganze Zahl aus dem Bereich von 2 bis 40 ist;
erhalten oder erhältlich nach dem Verfahren des 2. Aspektes. Wie bereits beim 2. Aspekt beschrieben, kann der Index "a" bei jeder der n Wiederholungseinheiten der allgemeinen Formel (III) gleich oder verschieden sein, d.h. bei m Wiederholungseinheiten gibt es maximal n verschiedene Werte für den Index "a". Ebenso kann "X" bei jeder der m Wiederholungseinheiten gleich oder verschieden sein, d.h. jede der n X-Gruppen ist unabhängig ausgewählt aus C(=O)-O-Gruppe bzw. -O-C(=O)-Gruppe und-O-C(=O)-O-Gruppe. X ist in den Oligomeren der allgemeinen Formel (III) nicht bei der letzten Wiederholungseinheit vorhanden. Dies bedeutet exemplarisch für m = 2 den folgenden Aufbau eines Oligomers der allgemeinen Formel (III):

Y¹-[(CH₂)ₐ-X-]-[(CH₂)ₐ-]-Y²

Der Aufbau für Oligomere der allgemeinen Formel (III) mit m im Bereich von 3 bis 40 ist analog.

Bevorzugt umfasst die Zusammensetzung weiterhin ein Monomer der allgemeinen Formel (IV)

R¹-O-C(=O)-O-R² (IV)

, wobei R¹, R² jeweils eine C1-C10-Alkylgruppe, bevorzugt eine C1-C5-Alkylgruppe, weiter bevorzugt eine C1- oder C2-Alkylgruppe, sind.

### 4. Aspekt - Verwendung für Re-Polymerisation

Gemäß eines vierten Aspektes betrifft die Erfindung die Verwendung der Zusammensetzung des dritten Aspektes zur Herstellung von Polymeren, insbesondere Polyethylen-artigen Polymeren umfassend Methylengruppen und mindestens eine funktionelle Gruppe ausgewählt aus O-C(=O)-Gruppe und O-C(=O)-O-Gruppe, wobei bevorzugt 90 bis 99,5 Mol-%, weiter bevorzugt 92 bis 98 Mol-%, des Polyethylen-artigen Polymers aus Methyleneinheiten (CH₂) bestehen.

Bevorzugt bezüglich der Verwendung weist das Polyethylen-artige Polymer eine Wiederholungseinheit der allgemeinen Formel (I)

[-X-(CH₂)ₐ-] (I)

auf, wobei
X ausgewählt ist aus C(=O)-O-Gruppe bzw. -O-C(=O)-Gruppe und-O-C(=O)-O-Gruppe;
a eine ganze Zahl aus dem Bereich von 8 bis 200 ist. In einer bevorzugten Ausführungsform ist die allgemeine Formel (I) wie folgt: [-X-(CH₂)ₐ-]ₙ, wobei "X" und "a" die angegebene Bedeutung haben. Wie bereits beim 2. Aspekt beschrieben, kann der Index "a" bei jeder der n Wiederholungseinheiten der allgemeinen Formel (III) gleich oder verschieden sein, d.h. bei n Wiederholungseinheiten gibt es maximal n verschiedene Werte für den Index "a". Ebenso kann "X" bei jeder der n Wiederholungseinheiten gleich oder verschieden sein, d.h. jede der n X-Gruppen ist unabhängig ausgewählt aus C(=O)-O-Gruppe bzw. -O-C(=O)-Gruppe und-O-C(=O)-O-Gruppe.

Das eingesetzte, bevorzugt Polyethylen-artige, Polymer weist ein zahlenmittleres Molekulargewicht (Mₙ) im Bereich von 5.000 bis 10.000.000 g/mol, bevorzugt im Bereich von 10.000 bis 10.000.000 g/mol auf. In bevorzugten Ausführungsformen weist es ein zahlenmittleres Molekulargewicht (Mₙ) im Bereich von 20.000 bis 10.000.000 g/mol oder im Bereich von 50.000 bis 10.000.000 g/mol, auf. Die Bestimmung von Mₙ erfolgt mittels Gelpermeationschromatographie (GPC) gegen Polystyrol-Standards im Bereich von 400 - 5.000.000 g/mol (universelle Kalibrierung). Details bezüglich des, bevorzugt Polyethylen-artigen, Polymers sind wie oben zum 2. Aspekt der Erfindung beschrieben; entsprechend wird hier auf alle zum 2. Aspekt der Erfindung beschriebenen Details zum Polymer Bezug genommen.

### 5. Aspekt - Verfahren zum Herstellen eines Fertigteils mittels Urformen

Die Erfindung betrifft gemäß einem fünften Aspekt ein Verfahren zum Herstellen eines Fertigteils. Das Fertigteil kann ein Bauteil einer Vorrichtung, wie beispielsweise ein Bauteil einer Maschine sein. Beispielsweise ist das Fertigteil ein Zahnrad. Das Verfahren umfasst Bereitstellen eines Materials, das ein Polyethylen-artiges Polymer aufweist. Das Polyethylen-artige Polymer umfasst Methylengruppen (CH₂) und mindestens eine funktionelle Gruppe ausgewählt aus O-C(=O)-Gruppe und O-C(=O)-O-Gruppe. Details bezüglich des, bevorzugt Polyethylen-artigen, Polymers sind wie oben zum 2. Aspekt der Erfindung beschrieben. Entsprechend wird hier auf alle zum 2. Aspekt der Erfindung beschriebene Details zum Polymer Bezug genommen. Das Fertigteil wird durch Urformen des so bereitgestellten Materials hergestellt.

Unter "Urformen" kann im Sinne der vorliegenden Erfindung insbesondere ein Fertigungsverfahren nach DIN 8580 verstanden werden, bei dem aus einem formlosen Stoff ein fester Körper hergestellt wird, der eine geometrisch definierte Form hat. Urformen wird genutzt, um die Erstform eines festen Körpers herzustellen und den Stoffzusammenhalt zu schaffen. Zum Urformen können Ausgangsstoffe prinzipiell im flüssigen, gasförmigen, plastischen, körnigen oder pulverförmigen Zustand genutzt werden, also mit unterschiedlichem rheologischen Verhalten.

Insbesondere umfasst das Urformen des Materials thermisches Urformen, d.h. Urformen bei gleichzeitiger Wärmezufuhr. Grundsätzlich ist im Rahmen der vorliegenden Erfindung das Urformen mindestens ein Verfahren ausgewählt aus der Gruppe bestehend aus: Spritzgießen, Extrudieren, Kalandrieren, Rotationsformen, Spritzblasformen.

Es wird explizit betont, dass mittels dieses Verfahrens jedes Fertigteil herstellbar ist, das mittels Urformen des beschriebenen Materials herstellbar ist.

### 6. Aspekt - Verfahren zum Herstellen eines Fertigteils mittels additiver Fertigung

Die Erfindung betrifft gemäß einem sechsten Aspekt ein Verfahren zum Herstellen eines Fertigteils aus einem Material, wobei das Material ein Polyethylen-artiges Polymer aufweist. Das Polyethylen-artige Polymer umfasst Methylengruppen (CH₂) und mindestens eine funktionelle Gruppe ausgewählt aus O-C(=O)-Gruppe und O-C(=O)-O-Gruppe. Details bezüglich des, bevorzugt Polyethylen-artigen, Polymers sind wie oben zum 2. Aspekt der Erfindung beschrieben. Entsprechend wird hier auf alle zum 2. Aspekt der Erfindung beschriebene Details zum Polymer Bezug genommen. Das Fertigteil wird mittels eines additiven Fertigungsverfahrens hergestellt.

Unter einem "additiven Fertigungsverfahren" kann im Sinne der vorliegenden Erfindung insbesondere ein Fertigungsverfahren verstanden werden, bei dem Material Schicht für Schicht aufgetragen und so dreidimensionale Gegenstände (Werkstücke) erzeugt werden. Dabei erfolgt der schichtweise Aufbau computergesteuert aus einem oder mehreren flüssigen oder festen Werkstoffen nach vorgegebenen Maßen und Formen. Beim Aufbau finden physikalische oder chemische Härtungs- oder Schmelzprozesse statt. Obwohl es sich oft um ein formendes Verfahren handelt, sind für ein konkretes Erzeugnis keine speziellen Werkzeuge wie zum Beispiel Gussformen erforderlich, die die jeweilige Geometrie des Werkstückes gespeichert haben. Das additive Fertigungsverfahren ist bevorzugt 3-D-Drucken.

Unter "3-D-Drucken" kann im Sinne der vorliegenden Erfindung insbesondere ein Fertigungsverfahren verstanden werden, bei dem Material Schicht für Schicht aufgetragen und so dreidimensionale Gegenstände (Werkstücke) erzeugt werden. Dabei erfolgt der schichtweise Aufbau computergesteuert aus einem oder mehreren flüssigen oder festen Werkstoffen nach vorgegebenen Maßen und Formen. Beim Aufbau finden physikalische oder chemische Härtungs- oder Schmelzprozesse statt. Obwohl es sich oft um ein formendes Verfahren handelt, sind für ein konkretes Erzeugnis keine speziellen Werkzeuge wie zum Beispiel Gussformen erforderlich, die die jeweilige Geometrie des Werkstückes gespeichert haben. Prinzipiell kann es sich um ein Verfahren gemäß aktuellem Normentwurf DIN EN ISO/ASTM 52900:2018 handeln und somit um Freistrahl-Bindemittelauftrag, Materialauftrag mit gerichteter Energieeinbringung, Materialextrusion, Freistrahl-Materialauftrag, pulverbettbasiertes Schmelzen, Schichtlaminierung oder badbasierte Photopolymerisation.

Bei dem Verfahren wird das Material als Filament bereitgestellt.

Unter "Filament" können im Sinne der vorliegenden Erfindung insbesondere thermoplastische Kunststoffe aus dem zuvor beschriebenen Material verstanden werden, die in Drahtform insbesondere auf Rollen konfektioniert im FDM/FFF-Verfahren (FDM - Fused Deposition Modeling; FFF - Fused Filament Fabrication) zum Einsatz kommen.

### BEISPIELE

Die Erfindung wird nachfolgend anhand von Beispielen erläutert, ohne sie auf diese zu beschränken.

### Beispiel 1 - Synthese von Polycarbonat-18

In einer typischen Polykondensationsreaktion zur Herstellung von PC-18, wurde ein mit 1,18-Octadecandiol (1 Equiv.) und einem Magnetrührer befülltes Dreihals-Schlenkrohr mittels Silikon-Septum verschlossen und für mindestens 1 Std. bei 60 °C unter Vakuum getrocknet. Im Anschluss an das Spülen des Aufbaus mit Stickstoff, wurde eine Kondensationsbrücke mit Rundkolben und Blasenzähler an das Schlenkrohr angebracht. Im Stickstoff-Gegenstrom wurde Diethylcarbonat (2,5 Equiv.) zugegeben und die Reaktionsmischung wurde auf 120 °C (Rühren bei 500 U/min) erhitzt bis sich ein homogenes Gemisch gebildet hatte. Im nächsten Schritt wurde eine Suspension von LiH (0,7 mol%) in Diethylcarbonat zugegeben. Nach ca. 15 - 45 min. begann das Auskondensieren von Ethanol über die Kondensationsbrücke. Die Reaktionsbedingungen wurden gehalten, bis sich kein Kondensat mehr bildete. Im nächsten Schritt wurde der Blasenzähler an der Kondensationsbrücke durch eine Membranpumpe ersetzt und die Oligomerisierungsreaktion wurde bei 180 °C unter Unterdruck (900 mbar bis 10 mbar) fortgesetzt. Im Anschluss an die Oligomerisierung wurde der Aufbau mit Stickstoff gespült, die Kondensationsbrücke durch einen Glasstopfen ersetzt und Hochvakuum (∼ 1-5 x 10⁻² mbar) über die Schlenkline angelegt. Auf Grund der zu diesem Zeitpunkt der Reaktion bereits erreichten hohen Viskosität der Polymerschmelze wurde der Magnetrührer abgeschalten. Die Polykondensationsreaktion wurde fortgeführt bis die Polymerschmelze auf Grund der hohen Viskosität nicht mehr fließfähig war. Um das entstandene Polymer in Lösung zu bringen, wurde im nächsten Schritt Xylol im Stickstoffgegenstrom bei 120 °C zugegeben. Nachdem die Polymerschmelze mit einem Spatel gelockert wurde, wurde der Magnetrührer wieder eingeschalten. Nach ca. 30 - 60 min. hatte sich eine homogene Lösung des Polymers gebildet, welche in kaltem Propan-2-ol ausgefällt wurde. Der gefällte Feststoff wurde wiederholt in Propan-2-ol gewaschen und schließlich über Nacht im Vakuumtrockenschrank getrocknet. Die Ausbeute betrug typischerweise über 95 mol% PC-18 bzgl. des eingesetzten 1,18-Octadecandiols. Das Polymer wurde durch ¹H- und ¹³C-NMR Spektroskopie, IR Spektroskopie, Gelpermeationschromatographie und Zug-Dehn-Experiment charakterisiert. Die mechanischen Eigenschaften des Polymers waren im typischen Bereich polyethylenartiger Materialien mit einer Bruchdehnung von 200 - 400% bei einem Elastizitätsmodul von 500 bis 1000 MPa.

### Beispiel 2 - Synthese von Polycarbonat-48

PC-48 wurde analog zur beschriebenen Herstellung von PC-18 gemäß Beispiel 1 aus 1,48-Octadecandiol synthetisiert, wobei 1,48-Octadecandiol mittels eines anderweitig publizierten katalytischen Prozesses aus Erucasäure hergestellt wurde. 1,48-Octadecandiol (10,0 g) wurden in einem Dreihals-Schlenkrohr mit Magnetrührer bei 100 °C getrocknet und anschließend mit Diethylcarbonat (8,5 ml, 5 Equiv.) und LiH (1,1 mg, 1 mol%) versetzt. Die Reaktionsmischung wurde analog zur Synthese von PC-18 oligomerisiert und im Anschluss bei 180 °C für 72 h polymerisiert um eine zur Schmelze von PC-18 vergleichbare Viskosität zu erreichen. Das entstandene Polymer wurde schließlich portionsweise in Xylol gelöst und in kaltem Propan-2-ol ausgefällt. Der gefällte Feststoff konnte mittels Filtration in quantitativer Ausbeute gewonnen werden (10,19 g, 98 %). Das Polymer wurde durch ¹H- und ¹³C-NMR Spektroskopie, IR Spektroskopie und Gelpermeationschromatographie charakterisiert.

### Beispiel 3 - Synthese von Polyester-18,18

In einer typischen Polykondensationsreaktion zur Herstellung von PE-18,18, wurde ein mit 1,18-Octadecandiol (1 Equiv.), 1,18-Dimethyloctadecandioat (1 Equiv.) und einem Magnetrührer befülltes Dreihals-Schlenkrohr mittels Silikon-Septum verschlossen und für mindestens 1 Std. bei 60 °C unter Vakuum getrocknet. Im Anschluss an das Spülen des Aufbaus mit Stickstoff, wurde eine Kondensationsbrücke mit Rundkolben und Blasenzähler an das Schlenkrohr angebracht. Im nächsten Schritt wurde im Stickstoff-Gegenstrom Titan(IV) butoxid (0,084 mol%) zur Reaktionsmischung zugegeben und die Temperatur wurde auf 180 °C erhöht (Rühren bei 350 U/min). Nach ca. 15 - 45 min. begann das Auskondensieren von Methanol über die Kondensationsbrücke. Die Reaktionsbedingungen wurden gehalten, bis sich kein Kondensat mehr bildete. Im nächsten Schritt wurde der Blasenzähler an der Kondensationsbrücke durch eine Membranpumpe ersetzt und die Oligomerisierungsreaktion wurde bei 180 °C unter Unterdruck (900 mbar bis 10 mbar) fortgesetzt. Im Anschluss an die Oligomerisierung wurde die Kondensationsbrücke durch einen Glasstopfen ersetzt und Hochvakuum (∼ 1-5 x 10⁻² mbar) über die Schlenkline angelegt. Auf Grund der zu diesem Zeitpunkt der Reaktion bereits erreichten hohen Viskosität der Polymerschmelze wurde der Magnetrührer abgeschalten. Die Polykondensationsreaktion wurde fortgeführt bis die Polymerschmelze auf Grund der hohen Viskosität nicht mehr fließfähig war. Um das entstandene Polymer in Lösung zu bringen, wurde im nächsten Schritt Xylol im Stickstoffgegenstrom bei 160 °C zugegeben. Nachdem die Polymerschmelze mit einem Spatel gelockert wurde, wurde der Magnetrührer wieder eingeschalten. Nach ca. 30 - 60 min. hatte sich eine homogene Lösung des Polymers gebildet, welche in kaltem Propan-2-ol ausgefällt wurde. Der gefällte Feststoff wurde wiederholt in Aceton gewaschen und schließlich über Nacht im Vakuumtrockenschrank getrocknet. Die Ausbeute betrug typischerweise über 95 mol% PE-18,18 bzgl. der Menge an eingesetztem Monomer. Das Polymer wurde durch ¹H- und ¹³C-NMR Spektroskopie, IR Spektroskopie und Gelpermeationschromatographie charakterisiert. Die mechanischen Eigenschaften des Polykondensats lagen im typischen Bereich Polyethylen-artiger Materialien mit einer Bruchdehnung von 350 - 500 % bei einem Elastizitätsmodul von 900 bis 1100 MPa.

### Beispiel 4 - Extrusion von 3D Druck Filament: Experimenteller Aufbau und Verfahren

Zur Herstellung von 3D Druck Filament im Labormaßstab wurde eine Düse verwendet, welche an einen Labor-Compounder Xplore MC-5 angeschlossen wurde und so die Extrusion von maßgenauem Filament der beschriebenen Polykondensate erlaubte. Die aus dem Compounder austretende Polymerschmelze wurde durch die Düse geleitet, kühlte hierbei ab und trat als formstabiles Filament aus der Düse aus. Typischerweise wurde zunächst die am Compounder angebrachte Düse für ca. 5 min. auf die Temperatur des Compounders vorgewärmt. Anschließend wurde das Filament mit den jeweiligen materialspezifischen Parametern extrudiert. Eine einzelne Compounder-Füllung erlaubte das Extrudieren von ca. 4 g Filament. Im semikontinuierlichen Betrieb (d.h. der Compounder wurde nachgefüllt während die Filament-Extrusion pausiert wurde) konnten, lediglich limitiert durch die Menge an vorhandenem Polymer, bis zu 23 g Filament an einem Stück extrudiert werden.

### Beispiel 5 - Extrusion von 3D Druck Filament: Prozessparameter

Zwei kommerziell erhältliche Farbstoffe (Omnidynamics MBR und MBB) sowie Kohlefaser (Durchmesser ∼ 7 µm ± 2, Länge: 200 µm ± 30) wurden beispielhaft für typische Polymeradditive gewählt, um daraus die entsprechenden Compounds mit PC-18 und PE-18,18 herzustellen. Zur Herstellung gefärbter Filamente wurden 4,85 g PC-18 oder PE-18,18 mit 150 mg (3 Gew.-%) Farbstoff bei 150 °C für mindestens 30 min. compoundiert und anschließend als Filament extrudiert. Zur Herstellung von kohlefaserverstärktem PC-18 wurden 4,5 g Polymer mit 500 mg Kohlefaser bei 170 °C für 30 min. compoundiert und anschließend als Filament extrudiert. Zur Herstellung von schwarzem PC-48 wurden 5 g Polymer mit 100 mg Kohlefaser bei 170 °C für 30 min. compoundiert und anschließend als Filament extrudiert. Die Durchmesser der mit dem beschriebenen Verfahren hergestellten Filamente bewegten sich im Bereich von 1,66 - 1,81 mm mit einer Standardabweichung von 0,01 - 0,02 mm (vgl. Durchmesser kommerzielles Filament: 1,75 ± 0,05 mm).

### Beispiel 6 - Depolymerisation von PC-18

Für die Depolymerisation von größeren Mengen von PC-18 wurde ein Stahlautoklav mit Glaseinsatz verwendet (300 ml nutzbares Innenvolumen). 20,0 g (64 mmol) PC-18 (schmelzprozessierte, grob zerkleinerte Stücke), 250 ml KOH basisches EtOH (10 Gewichts% bzgl. PC-18) und ein Magnetrührfisch wurden hierzu in den Glaseinsatz gefüllt. Der Autoklav wurde auf einer Heizplatte auf 120 °C erhitzt und bei 500 Umdrehungen pro Minute magnetisch gerührt. Nach der typischen Depolymerisationszeit von 1 bis 24 h wurde der Heizer ausgeschalten und der Autoklav wurde auf 40 °C abgekühlt. Die warme Lösung wurde quantitativ mit EtOH in ein Becherglas gespült. Die Mischung wurde eingedampft und anschließend in MeOH umkristallisiert. Die Temperatur des Gemischs bei der Filtration betrug - 30°C um eine quantitative Abtrennung zu gewährleisten. Die abfiltrierten Feststoffe wurden getrocknet und 17,91 g, entsprechend 98 mol% (bezogen auf eingesetztes PC-18), von 1,18-Octadecandiol wurden dabei wiedergewonnen. Die Reinheit von > 99% wurde per Gaschromatographie (FID) bestimmt.

### Beispiel 7 - Abtrennung von PC-18 von Polyolefinen durch Depolymerisation

Um die Abtrennung von PC-18 aus einem typischen Plastikmüllgemisch von Polyolefinen, wie sie bei der industriellen Müllsortierung anfallen, zu simulieren, wurde ein blau gefärbtes Stück eines 3D-gedruckten PC-18 Stücks mit jeweils einem gefärbten Stück kommerziellen Polypropylens als auch Polyethylens vermischt und gemeinsam in einem Glaseinsatz für Druckreaktoren mit KOH basischem MeOH (10 Gewichts% bzgl. PC-18) depolymerisiert (100 °C, 500 Umdrehungen pro Minute für 24 h um vollständigen Umsatz zu gewährleisten). Die Vollständigkeit der Reaktion wurde per ¹H-NMR Spektroskopie nachgewiesen. Die beiden verbleibenden Polyolefinstücke wurden nach dem Experiment mit MeOH gewaschen und getrocknet. Die beiden Polyolefine wiesen dabei keinen Gewichtsverlust auf, außerdem war die Farbe der Kunststoffe selbst unverändert, jedoch befanden sich blaue Flecken auf der Oberfläche durch den abgelagerten Farbstoff des gefärbten PC-18. Die Filtrate der Reaktion wurden kombiniert und zur Trocknung eingedampft. Das farblose Monomer 1,18-Octadecandiol wurde nach Umkristallisation aus MeOH dabei nahezu quantitativ zu 180,1 mg, entsprechend 98 mol% (bezogen auf das eingesetzte PC-18), wiedergewonnen. Die Reinheit von > 99% wurde mit Gaschromatographie (FID) bestimmt.

### Beispiel 8 - Depolymerisation von PE-18,18

Um den Reaktionsfortschritt verfolgen zu können, wurde die Depolymerisation von PE-18,18 (25,0 g ungefärbtes Polymer oder 5,0 g mittels Omnidynamics MBR gefärbtes Polymer) in einem Glasrohrautoklav (500 ml Volumen) durchgeführt. In einem typischen Depolymerisationsexperiment wurde das Glasrohr mit einem starken Lanthanoid-Magnetrührfisch, schmelzprozessiertem und vorgeschnittenem PE-18,18 sowie Methanol (16 ml pro 1 g PE-18,18) befüllt und in einem Heizblock temperiert (120 °C bei 200 U/min).
Nach einer typischen Depolymerisationszeit von 2 bis 24 h wurde das Reaktionsgemisch quantitativ in einen Rundkolben überführt und anschließend das Lösemittel Methanol unter Vakuum entfernt.

Diese Methode lieferte eine farblose, stöchiometrisch exakte 1:1 Mischung der beiden eingesetzten Monomere (1,18-Octadecandiol und 1,18-Dimethyloctadecandioat), was durch ¹H-NMR-Experimente nachgewiesen werden konnte. Die Ausbeute war quantitativ (27,6 g der 1:1 Monomeren-Mischung aus 25,0 g PE-18,18).

Erfolgreich verlaufende, analoge Depolymerisationsexperimente mit PE-18,18 wurden im kleinen Maßstab (200 mg Polymer) ebenfalls bei höheren Temperaturen (z.B. 150 °C) sowie mit Katalysator (z.B. 10 Gew.-% KOH) in Metallautoklaven durchgeführt.

### Beispiel 9 - Repolymerisation zu PE-18,18

Die 1:1 Monomeren-Mischung aus Beispiel 8 konnte ohne Aufreinigung analog zur beschriebenen Herstellung von "virgin" PE-18,18 aus Beispiel 3 zu gleichwertigem Polymer (d.h. keine Verschlechterung der mechanischen und chemischen Eigenschaften des Materials, wie u.a. durch Zug-Dehn-Versuche, ¹H-NMR und Gel-Permeations-Chromatographie gezeigt werden konnte) repolymerisiert werden.

### Beispiel 10 - Depolymerisation von PE-18,18 mit Verunreinigungen

Zur Simulation einer Verunreinigung wurde dem PE-18,18 schwarz gefärbtes Polypropylen - als Beispiel für einen weitverbreiteten kommerziellen Kunststoff- zugesetzt und eine Depolymerisation wie in Beispiel 8 durchgeführt. Das schwarz gefärbte Polypropylen war nach dem Prozess unverändert und konnte einfach mittels Pinzette aus der farblosen Monomeren-Mischung entfernt werden.

Im Falle der Depolymerisation von mittels Omnidynamics MBR gefärbtem PE-18,18 wurde das Reaktionsgemisch nach der Depolymerisation in einen Rundkolben überführt, wobei der eingesetzte Farbstoff im Glasrohrautoklav zurückblieb (als ganzes Stück am Boden des Reaktors klebend und theoretisch wiederverwendbar) und somit effektiv und einfach abgetrennt werden konnte. Die vollständige Abtrennung des Farbstoffes konnte durch ¹H-NMR Experimente nachgewiesen werden. Diese zeigten, dass wieder eine repolymerisierbare, stöchiometrisch exakte 1:1 Mischung der beiden eingesetzten Monomere erhalten wurde.

### Beispiel 11 - 3D-Druck mittels extrudiertem Filament

Für den 3D-Druck der beschriebenen Polykondensate wurde ein handelsüblicher, nicht modifizierter Drucker der Marke Prusa mit der Modellbezeichnung Prusa i3 MK3 verwendet.

Dieser war mit einem Druckbett aus Edelstahl, überzogen mit einer Polyetherimidfolie (PEI), ausgestattet.

### 11.1 Zug-Dehn Prüfkörper nach ISO 527-2, Typ 5A

Zug-Dehn Prüfkörper nach ISO 527-2-5A wurden von allen beschriebenen Polymeren mit folgenden Einstellungen hergestellt:

| | |
|---|---|
| • Düsendurchmesser: | 0.8 mm |
| • Düsentemperatur: | 230 °C |
| • Druckbetttemp.: | 100 °C |
| • Druckgeschwindigkeit: | 5 mm/s |
| • Schichthöhe: | 0.4 mm |
| • allg. Extrusionsbreite: | 0.8 mm |
| • Kühlung durch Lüfter: | komplett deaktiviert |

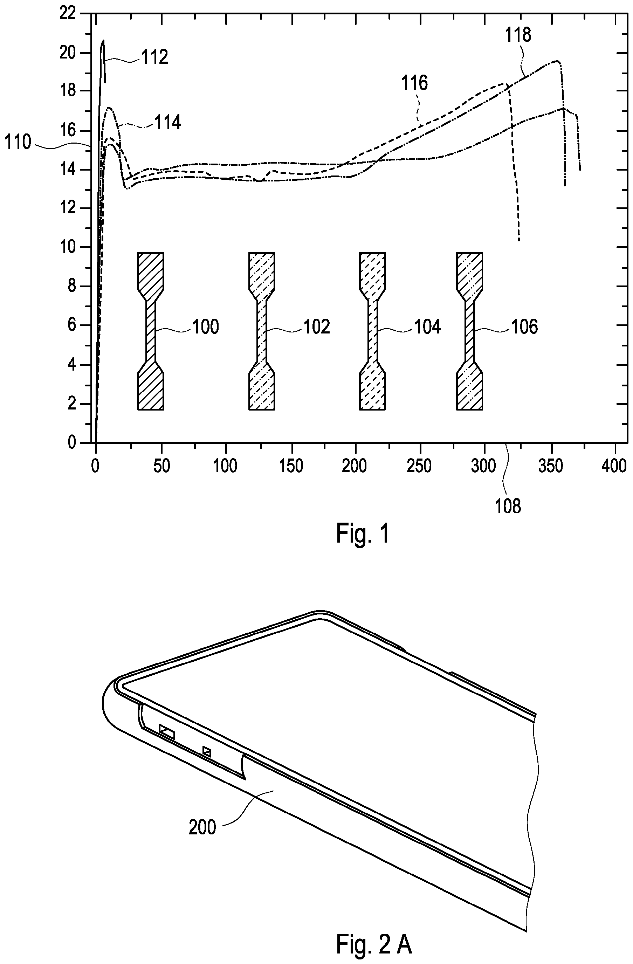

Figur 1 zeigt ein Spannungs-Dehnungs-Diagramm für Zug-Dehn Prüfkörper 100, 102, 104, 106 nach ISO 527-2-5A hergestellt aus PC-18, die mittels des Druckers mit den angegebenen Parametern hergestellt wurden. Als Ausgangsmaterialien für die in Figur 1 beispielhaft dargestellten Messergebnisse wurden die gemäß Beispiel 4 und Tabelle 1 genannten PC-18 Filamente verwendet. Auf der X-Achse 108 ist die Dehnung in % angegeben. Auf der Y-Achse 110 ist die Spannung in MPa angegeben. Die Kurven stellen aus Gründen der Übersichtlichkeit jeweils nur das Ergebnis einer Messung nach ISO 527-2-5A dar, die innerhalb der Schwankung aller untersuchten Prüfkörper liegen. In Figur 1 gibt die Kurve 112 das Messergebnis für einen weißen Prüfkörper 100 an, gibt die Kurve 114 das Messergebnis für einen roten Prüfkörper 102 an, gibt die Kurve 116 das Messergebnis für einen blauen Prüfkörper 104 an und gibt die Kurve 118 das Messergebnis eines kohlefaserverstärkten Blends als Material für den Prüfkörper 106 zum Vergleich an.

Die mechanischen Eigenschaften der aus PE-18,18 und PC-18 mittels 3D-Druck hergestellten Zug-Dehn Prüfkörper 100, 102, 104, 106 (ISO 527-2-5A) entsprechen den Polyethylen-artigen Eigenschaften der mittels Spritzguss aus denselben Polymeren hergestellten Prüfkörper. Mit den in diesem Patent beschriebenen Polymeren lassen sich somit HDPE-artige Eigenschaften sowohl mittels Spritzguss als auch 3D-Druck realisieren.

### 11.2 Schutzhülle für Smartphone (passend für iPhone 11 Pro) aus PE-18,18

Die Druckparameter zur Herstellung einer Schutzhülle 200 für ein Smartphone aus PE-18,18 sind im Folgenden angegeben. Es handelt sich um den Auszug der relevanten Parameter aus dem verwendeten G-Code zur Herstellung des Objekts:
; external perimeters extrusion width = 0.45mm
; perimeters extrusion width = 0.45mm
; infill extrusion width = 0.45mm
; solid infill extrusion width = 0.45mm
; top infill extrusion width = 0.40mm
; first layer extrusion width = 0.42mm
M201 X1000 Y1000 Z1000 E5000 ; sets maximum accelerations, mm/sec^2
M203 X200 Y200 Z12 E120 ; sets maximum feedrates, mm/sec
M204 P1250 R1250 T1250 ; sets acceleration (P, T) and retract acceleration (R), mm/sec^2
M205 X8.00 Y8.00 Z0.40 E1.50 ; sets the jerk limits, mm/sec
M104 S200 ; set extruder temp
M140 S95 ; set bed temp
M190 S95 ; wait for bed temp
M109 S200 ; wait for extruder temp
; estimated printing time (normal mode) = 1h 0m 37s
; avoid_crossing_perimeters = 1
; bed_shape = 0x0,250x0,250x210,0x210
; bed_temperature = 95
; bridge_acceleration = 1000
; bridge_fan_speed = 50
; brim_width = 10
; colorprint_heights =
; complete_objects = 0
; cooling = 0
; cooling_tube_length = 5
; cooling_tube_retraction = 91.5
; default_acceleration = 1000
; deretract_speed = 0
; disable_fan_first_layers = 4
; duplicate_distance = 6
; extra_loading_move = -2
; extruder_clearance_height = 20
; extruder_clearance_radius = 20
; extruder_colour = #FFFF00
; extruder_offset = 0x0
; extrusion_axis = E
; extrusion_multiplier = 0.97
; fan_always_on = 0
; fan_below_layer_time = 100
; filament_colour = #DEE0E6
; filament_cooling_final_speed = 3.4
; filament_cooling_initial_speed = 2.2
; filament_cooling_moves = 4
; filament_cost = 72
; filament_density = 0.89
; filament_diameter = 1.6
; filament_load_time = 0
; filament_loading_speed = 28
; filament_loading_speed_start = 3
; filament_max_volumetric_speed = 5
; filament_minimal_purge_on_wipe_tower = 15
; filament_soluble = 0
; filament_toolchange_delay = 0
; filament_type = PP
; filament_unload_time = 0
; filament_unloading_speed = 90
; filament_unloading_speed_start = 100
; first_layer_acceleration = 1000
; first_layer_bed_temperature = 95
; first_layer_extrusion_width = 0.42
; first_layer_speed = 30
; first_layer_temperature = 200
; gcode_comments = 0
; gcode_flavor = marlin
; gcode_label_objects = 0
; high_current_on_filament_swap = 0
; infill_acceleration = 1250
; infill_first = 0
; machine_max_acceleration_e = 5000,5000
; machine_max_acceleration_extruding = 1250,1250
; machine_max_acceleration_retracting = 1250,1250
; machine_max_acceleration_x = 1000,960
; machine_max_acceleration_y = 1000,960
; machine_max_acceleration_z = 1000,1000
; machine_max_feedrate_e = 120,120
; machine_max_feedrate_x = 200,100
; machine_max_feedrate_y = 200,100
; machine_max_feedrate_z = 12,12
; machine_maxjerk_e = 1.5,1.5
; machine_maxjerk_x = 8,8
; machine_maxjerk_y = 8,8
; machine_maxjerk_z = 0.4,0.4
; machine_min_extruding_rate = 0,0
; machine_min_travel_rate = 0,0
; max_fan_speed = 30
; max_layer_height = 0.25
; max_print_height = 210
; max_print_speed = 200
; max_volumetric_speed = 0
; min_fan_speed = 30
; min_layer_height = 0.07
; min_print_speed = 15
; min_skirt_length = 4
; notes =
; nozzle_diameter = 0.4
; only_retract_when_crossing_perimeters = 0
; ooze_prevention = 0
; parking_pos_retraction = 92
; perimeter_acceleration = 800
; post_process =
; printer_model = MK3
; resolution = 0
; retract_before_travel = 1
; retract_before_wipe = 0%
; retract_layer_change = 1
; retract_length = 1
; retract_length_toolchange = 4
; retract_lift = 0.4
; retract_lift_above = 0
; retract_lift_below = 209
; retract_restart_extra = 0
; retract_restart_extra_toolchange = 0
; retract_speed = 20
; silent_mode = 1
; single_extruder_multi_material = 0
; single_extruder_multi_material_priming = 0
; skirt_distance = 2
; skirt_height = 1
; skirts = 0
; slowdown_below_layer_time = 20
; spiral_vase = 0
; standby_temperature_delta = -5
; temperature = 200
; threads = 4
; toolchange_gcode =
; travel_speed = 180
; use_firmware_retraction = 0
; use_relative_e_distances = 1
; use_volumetric_e = 0
; variable_layer_height = 1
; wipe = 0
; wipe_tower = 1
; wipe_tower_bridging = 10
; wipe_tower_rotation_angle = 0
; wipe_tower_width = 60
; wipe_tower_x = 170
; wipe_tower_y = 125
; wiping_volumes_extruders = 70,70
; wiping_volumes_matrix = 0
; z_offset = 0
; clip_multipart_objects = 1
; dont_support_bridges = 1
; elefant_foot_compensation = 0
; extrusion_width = 0.45
; first_layer_height = 0.2
; infill_only_where_needed = 0
; interface_shells = 0
; layer_height = 0.2
; raft_layers = 0
; seam_position = nearest
; slice_closing_radius = 0.049
; support_material = 0
; support_material_angle = 0
; support_material_auto = 1
; support_material_buildplate_only = 0
; support_material_contact_distance = 0.1
; support_material_enforce_layers = 0
; support_material_extruder = 0
; support_material_extrusion_width = 0.35
; support_material_interface contact_loops = 0
; support_material_interface_extruder = 0
; support_material_interface_layers = 2
; support_material_interface_spacing = 0.2
; support_material_interface_speed = 100%
; support_material_pattern = rectilinear
; support_material_spacing = 2
; support_material_speed = 40
; support_material_synchronize_layers = 0
; support_material_threshold = 55
; support_material_with_sheath = 0
; support_material_xy_spacing = 50%
; wipe_into_objects = 0
; xy_size_compensation = 0
; bottom_fill_pattern = rectilinear
; bottom_solid_layers = 4
; bridge_angle = 0
; bridge_flow_ratio = 0.95
; bridge_speed = 40
; ensure_vertical_shell_thickness = 1
; external_perimeter_extrusion_width = 0.45
; external_perimeter_speed = 40
; external_perimeters_first = 0
; extra_perimeters = 0
; fill_angle = 45
; fill_density = 100%
; fill_pattern = rectilinear
; gap_fill_speed = 40
; infill_every_layers = 1
; infill_extruder = 1
; infill_extrusion_width = 0.45
; infill_overlap = 50%
; infill_speed = 40
; overhangs = 0
; perimeter_extruder = 1
; perimeter_extrusion_width = 0.45
; perimeter_speed = 40
; perimeters = 2
; small_perimeter_speed = 40
; solid_infill_below_area = 0
; solid_infill_every_layers = 0
; solid_infill_extruder = 1
; solid_infill_extrusion_width = 0.45
; solid_infill_speed = 40
; thin_walls = 0
; top_fill_pattern = rectilinear
; top_infill_extrusion_width = 0.4
; top_solid_infill_speed = 40
; top_solid_layers = 5
; wipe_into_infill = 0
; print_settings_id = 0.20mm PE-18,18
; filament_settings_id = PE-18,18
; printer_settings_id = Original Prusa i3 MK3 PE-18,18
; printer_model = MK3
; printer_variant = 0.4

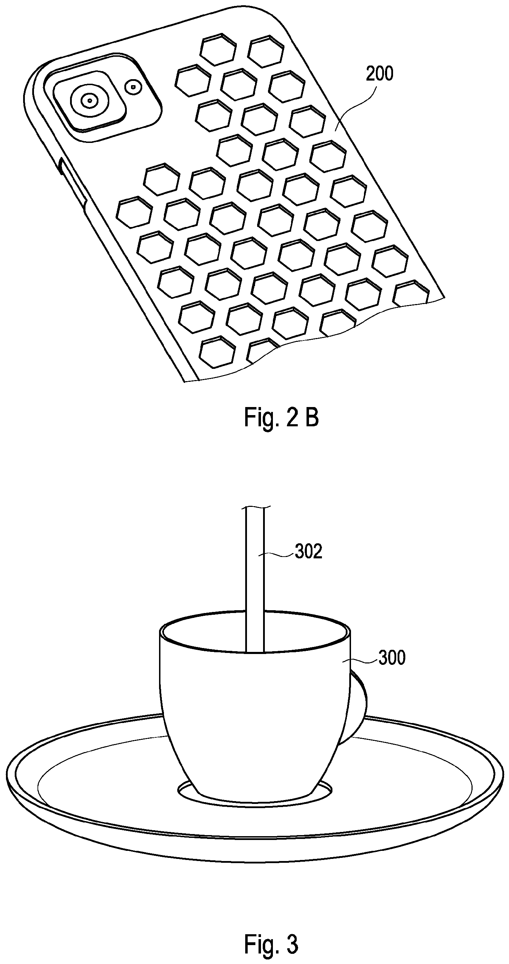

Abbildung 2A zeigt die Vorderansicht und Abbildung 2B die entsprechende Rückansicht der aus PE-18,18 hergestellten Smartphone-Schutzhülle 200. Wie aus den Figuren 2A und 2B ersichtlich ist, lässt sich mit dem Material PE-18,18 eine sehr gute Druckbarkeit erreichen, was die (additive) Fertigung auch von komplexen Objekten ermöglicht.

### 11.3 Gefärbte Espressotasse aus PC-48

Die Druckparameter zur Herstellung einer kleinen Tasse 300 aus gefärbtem PC-48 sind im Folgenden angegeben. Es handelt sich um den Auszug der relevanten Parameter aus dem verwendeten G-Code zur Herstellung des Objekts:
; external perimeters extrusion width = 0.45mm
; perimeters extrusion width = 0.45mm
; infill extrusion width = 0.45mm
; solid infill extrusion width = 0.45mm
; top infill extrusion width = 0.40mm
; first layer extrusion width = 0.42mm

M201 X1000 Y1000 Z1000 E5000 ; sets maximum accelerations, mm/sec^2
M203 X200 Y200 Z12 E120 ; sets maximum feedrates, mm/sec
M204 P1250 R1250 T1250 ; sets acceleration (P, T) and retract acceleration (R), mm/sec^2
M205 X8.00 Y8.00 Z0.40 E1.50 ; sets the jerk limits, mm/sec
M205 S0 T0 ; sets the minimum extruding and travel feed rate, mm/sec
M104 S250 ; set extruder temp
M140 S100 ; set bed temp
M190 S100 ; wait for bed temp
M109 S250 ; wait for extruder temp
; estimated printing time (normal mode) = 1h 48m 3s
; avoid_crossing_perimeters = 0
; bed_shape = 0x0,250x0,250x210,0x210
; bed_temperature = 100
; between_objects_gcode =
; bridge_acceleration = 1000
; bridge_fan_speed = 0
; brim_width = 0
; colorprint_heights =
; complete_objects = 0
; cooling = 0
; cooling_tube_length = 5
; cooling_tube_retraction = 91.5
; default_acceleration = 1000
; deretract_speed = 0
; disable_fan_first_layers = 3
; duplicate_distance = 6
; end_filament_gcode = "; Filament-specific end gcode"
; extra_loading_move = -2
; extruder_clearance_height = 20
; extruder_clearance_radius = 20
; extruder_colour = #FFFF00
; extruder_offset = 0x0
; extrusion_axis = E
; extrusion_multiplier = 0.9
; fan_always_on = 1
; fan_below_layer_time = 10
; filament_colour = #FF8000
; filament_cooling_final_speed = 3.4
; filament_cooling_initial_speed = 2.2
; filament_cooling_moves = 4
; filament_cost = 21.2
; filament_density = 1.2
; filament_diameter = 1.65
; filament_load_time = 0
; filament_loading_speed = 28
; filament_loading_speed_start = 3
; filament_max_volumetric_speed = 8
; filament_minimal_purge_on_wipe_tower = 15
; filament_notes = ""
; filament_soluble = 0
; filament_toolchange_delay = 0
; filament_type = PLA
; filament_unload_time = 0
; filament_unloading_speed = 90
; filament_unloading_speed_start = 100
; first_layer_acceleration = 1000
; first_layer_bed_temperature = 100
; first_layer_extrusion_width = 0.42
; first_layer_speed = 5
; first_layer_temperature = 250
; gcode_comments = 0
; gcode_flavor = marlin
; gcode_label_objects = 0
; high_current_on_filament_swap = 0
; infill_acceleration = 1250
; infill_first = 0
; machine_max_acceleration_e = 5000,5000
; machine_max_acceleration_extruding = 1250,1250
; machine_max_acceleration_retracting = 1250,1250
; machine_max_acceleration_x = 1000,960
; machine_max_acceleration_y = 1000,960
; machine_max_acceleration_z = 1000,1000
; machine_max_feedrate_e = 120,120
; machine_max_feedrate_x = 200,100
; machine_max_feedrate_y = 200,100
; machine_max_feedrate_z = 12,12
; machine_maxjerk_e = 1.5,1.5
; machine_maxjerk_x = 8,8
; machine_maxjerk_y = 8,8
; machine_maxjerk_z = 0.4,0.4
; machine_min_extruding_rate = 0,0
; machine_min_travel_rate = 0,0
; max_fan_speed = 30
; max_layer_height = 0.25
; max_print_height = 210
; max_print_speed = 200
; max_volumetric_speed = 0
; min_fan_speed = 30
; min_layer_height = 0.07
; min_print_speed = 15
; min_skirt_length = 4
; notes =
; nozzle_diameter = 0.4
; only_retract_when_crossing_perimeters = 0
; ooze_prevention = 0
; parking_pos_retraction = 92
; perimeter_acceleration = 800
; post_process =
; printer_model = MK3
; remaining_times = 1
; resolution = 0
; retract_before_travel = 1
; retract_before_wipe = 0%
; retract_layer_change = 1
; retract_length = 0
; retract_length_toolchange = 4
; retract_lift = 0.6
; retract_lift_above = 0
; retract_lift_below = 209
; retract_restart_extra = 0
; retract_restart_extra_toolchange = 0
; retract_speed = 35
; silent_mode = 1
; single_extruder_multi_material = 0
; single_extruder_multi_material_priming = 0
; skirt_distance = 2
; skirt_height = 1
; skirts = 1
; slowdown_below_layer_time = 20
; spiral_vase = 0
; standby_temperature_delta = -5
; temperature = 250
; threads = 4
; toolchange_gcode =
; travel_speed = 180
; use_firmware_retraction = 0
; use_relative_e_distances = 1
; use_volumetric_e = 0
; variable_layer_height = 1
; wipe = 0
; wipe_tower = 1
; wipe_tower_bridging = 10
; wipe_tower_rotation_angle = 0
; wipe_tower_width = 60
; wipe_tower_x = 170
; wipe_tower_y = 125
; wiping_volumes_extruders = 70,70
; wiping_volumes_matrix = 0
; z_offset = 0
; clip_multipart_objects = 1
; dont_support_bridges = 1
; elefant_foot_compensation = 0
; extrusion_width = 0.45
; first_layer_height = 0.2
; infill_only_where_needed = 0
; interface_shells = 0
; layer_height = 0.2
; raft_layers = 0
; seam_position = nearest
; slice_closing_radius = 0.049
; support_material = 0
; support_material_angle = 0
; support_material_auto = 1
; support_material_buildplate_only = 0
; support_material_contact_distance = 0.1
; support_material_enforce_layers = 0
; support_material_extruder = 0
; support_material_extrusion_width = 0.35
; support_material_interface_contact_loops = 0
; support_material_interface_extruder = 0
; support_material_interface_layers = 2
; support_material_interface_spacing = 0.2
; support_material_interface_speed = 100%
; support_material_pattern = rectilinear
; support_material_spacing = 2
; support_material_speed = 5
; support_material_synchronize_layers = 0
; support_material_threshold = 55
; support_material_with_sheath = 0
; support_material_xy-spacing = 50%
; wipe_into_objects = 0
; xy_size_compensation = 0
; bottom_fill_pattern = rectilinear
; bottom_solid_layers = 3
; bridge_angle = 0
; bridge_flow_ratio = 0.95
; bridge_speed = 5
; ensure_vertical_shell_thickness = 1
; external_perimeter_extrusion_width = 0.45
; external_perimeter_speed = 5
; external_perimeters_first = 0
; extra_perimeters = 0
; fill_angle = 45
; fill_density = 0%
; fill_pattern = grid
; gap_fill_speed = 40
; infill_every_layers = 1
; infill_extruder = 1
; infill_extrusion_width = 0.45
; infill_overlap = 40%
; infill_speed = 10
; overhangs = 0
; perimeter_extruder = 1
; perimeter_extrusion_width = 0.45
; perimeter_speed = 5
; perimeters = 1
; small_perimeter_speed = 5
; solid_infill_below_area = 0
; solid_infill_every_layers = 0
; solid_infill_extruder = 1
; solid_infill_extrusion_width = 0.45
; solid_infill_speed = 10
; thin_walls = 1
; top_fill_pattern = rectilinear
; top_infill_extrusion_width = 0.4
; top_solid_infill_speed = 50
; top_solid_layers = 0
; wipe_into_infill = 0
; print_settings_id = 0.20mm Vase Mode PC48 CUP
; filament_settings_id = "PC48 240_100_NOFAN"
; printer_settings_id = Original Prusa i3 MK3 0.4mm NORETRACT
; printer_model = MK3
; printer_variant = 0.4

Figur 3 zeigt eine perspektivische Ansicht der so hergestellten Tasse 300. Als thermischer Stresstest wurde die Tasse mit Wasser 302 mit einer Temperatur von mindestens 95 °C befüllt und optisch auf Formveränderungen untersucht. Zum Vergleich wurde eine Tasse gleicher Form aus Polylactiden (PLA) hergestellt (nicht näher gezeigt) und ebenfalls mit Wasser mit einer Temperatur von mindestens 95 °C befüllt. Bei der aus PC48 hergestellten Tasse konnte mit bloßem Auge nach Befüllen mit dem Wasser keine Formveränderung festgestellt werden. Dahingegen hat sich die Tasse hergestellt aus PLA deutlich verformt. Dies zeigt, dass das Material PC-48 im Vergleich zu PLA thermisch robuster ist.

## Patentansprüche

1. Verfahren zur Herstellung von Monomeren der allgemeinen Formel (II) und/oder Oligomeren der allgemeinen Formel (III)
Y¹-(CH₂)_{c}-Y² (II)
, wobei Y¹ und Y² jeweils unabhängig voneinander ausgewählt sind aus R-O-C(=O)-Gruppe bzw. -C(=O)-O-R-Gruppe und OH-Gruppe, wobei R ein Wasserstoffatom oder eine C1-C10-Alkylgruppe ist; und der Index c eine ganze Zahl aus dem Bereich von 8 bis 200 ist,
Y¹-[(CH₂)ₐ-X-]ₘ-Y² (III)
, wobei Y¹ und Y² jeweils unabhängig voneinander ausgewählt sind aus R-O-C(=O)-O-Gruppe bzw. -O-C(=O)-O-R-Gruppe, R-O-C(=O)-Gruppe bzw. -C(=O)-O-R-Gruppe und OH-Gruppe, wobei R ein Wasserstoffatom oder eine C1-C10-Alkylgruppe ist; X bei jeder der m Wiederholungseinheiten unabhängig ausgewählt ist aus C(=O)-O-Gruppe bzw. -O-C(=O)-Gruppe und-O-C(=O)-O-Gruppe, wobei X in der letzten Wiederholungseinheit von Formel (III) nicht vorhanden ist; a eine ganze Zahl aus dem Bereich von 8 bis 200 ist, wobei a bei jeder der m Wiederholungseinheiten gleich oder verschieden ist; und der Index m eine ganze Zahl aus dem Bereich von 2 bis 40 ist;
wobei das Verfahren umfasst:
i) Erzeugen einer alkoholischen und/oder wässrigen Mischung eines Polyethylen-artigen Polymers umfassend Methylengruppen und mindestens eine funktionelle Gruppe ausgewählt aus O-C(=O)-Gruppe und O-C(=O)-O-Gruppe;
ii) Erhitzen der gemäß i) bereitgestellten Mischung auf eine Temperatur im Bereich von 20 bis 400 °C;
iii) Abtrennen der Monomere der allgemeinen Formel (II) und/oder Oligomere der allgemeinen Formel (III) aus bzw. von der gemäß ii) erhaltenen alkoholischen und/oder wässrigen Mischung.

2. Verfahren nach Anspruch 1, weiterhin umfassend:
iv) optionale Aufreinigung der gemäß iii) erhaltenen Monomere der allgemeinen Formel (II) und/oder Oligomere der allgemeinen Formel (III);
v) Repolymerisation der gemäß iii) oder iv) erhaltenen Monomere der allgemeinen Formel (II) und/oder Oligomere der allgemeinen Formel (III) zu einem Polymer.

3. Verfahren zur Herstellung von Monomeren der allgemeinen Formel (II) und/oder Oligomeren der allgemeinen Formel (III) nach einem der Ansprüche 1 oder 2, wobei das Polyethylen-artige Polymer umfassend Methylengruppen und mindestens eine funktionelle Gruppe ausgewählt aus O-C(=O)-Gruppe und O-C(=O)-O-Gruppe eine Wiederholungseinheit der allgemeinen Formel (I) aufweist
[-X-(CH₂)ₐ-] (I)
, wobei
X ausgewählt ist aus C(=O)-O-Gruppe bzw. -O-C(=O)-Gruppe und -O-C(=O)-O-Gruppe;
a eine ganze Zahl aus dem Bereich von 8 bis 200 ist.

4. Verfahren zur Herstellung von Monomeren der allgemeinen Formel (II) und/oder Oligomeren der allgemeinen Formel (III) nach einem der Ansprüche 1 bis 3, wobei der Alkohol ausgewählt ist aus der Gruppe der monofunktionellen oder polyfunktionellen C1-C10-Alkohole und deren Mischungen, wobei die alkoholische Mischung des Polyethylen-artigen Polymers gemäß i) einen Wassergehalt im Bereich von 0 bis 5 Gewichts-% bezogen auf das Gesamtgewicht der alkoholischen Mischung aufweist; oder wobei für die Erzeugung der Mischung gemäß (i) Wasser verwendet wird.

5. Verfahren zur Herstellung von Monomeren der allgemeinen Formel (II) und/oder Oligomeren der allgemeinen Formel (III) nach einem der Ansprüche 1 bis 4, wobei die in ii) erhaltene alkoholische und/oder wässrige Lösung Mischung umfassend Monomere der allgemeinen Formel (II) und/oder Oligomere der allgemeinen Formel (III) weiterhin Monomere der allgemeinen Formel (IV) umfasst
R¹-O-C(=O)-O-R² (IV)
wobei
R¹, R² jeweils C1-C10-Alkylgruppen sind.

6. Zusammensetzung umfassend ein Monomer der allgemeinen Formel (II) und/oder ein Oligomer der allgemeinen Formel (III)
Y¹-(CH₂)_{c}-Y² (II)
wobei Y¹ und Y² jeweils unabhängig voneinander ausgewählt sind aus R-O-C(=O)-Gruppe bzw. -C(=O)-O-R-Gruppe und OH-Gruppe, wobei R ein Wasserstoffatom oder eine C1-C10-Alkylgruppe ist; und der Index c eine ganze Zahl aus dem Bereich von 8 bis 200 ist,
Y¹-[(CH₂)ₐ-X-]ₘ-Y² (III)
wobei
Y¹ und Y² jeweils unabhängig voneinander ausgewählt sind aus R-O-C(=O)-O-Gruppe bzw. -O-C(=O)-O-R-Gruppe, R-O-C(=O)-Gruppe bzw. -C(=O)-O-R-Gruppe und OH-Gruppe, wobei R ein Wasserstoffatom oder eine C1-C10-Alkylgruppe ist; X bei jeder der m Wiederholungseinheiten unabhängig ausgewählt ist aus C(=O)-O-Gruppe bzw. -O-C(=O)-Gruppe und-O-C(=O)-O-Gruppe; a eine ganze Zahl aus dem Bereich von 8 bis 200 ist, wobei a bei jeder der m Wiederholungseinheiten gleich oder verschieden ist; und der Index m eine ganze Zahl aus dem Bereich von 2 bis 40 ist;
erhalten oder erhältlich nach einem Verfahren gemäß einem der Ansprüche 1 bis 5.

7. Verwendung der Zusammensetzung gemäß Anspruch 6 zur Herstellung von Polymeren, insbesondere Polyethylen-artigen Polymeren umfassend Methylengruppen und mindestens eine funktionale funktionelle Gruppe ausgewählt aus O-C(=O)-Gruppe und O-C(=O)-O-Gruppe, wobei bevorzugt 90 bis 99,5 Mol-%, weiter bevorzugt 92 bis 98 Mol-%, des Polyethylen-artigen Polymers aus Methyleneinheiten (CH₂) bestehen.

8. Verfahren zum Herstellen eines Fertigteils, umfassend Bereitstellen eines Materials, das ein Polyethylen-artiges Polymer aufweist, wobei das Polyethylen-artige Polymer Methylengruppen (CH₂) und mindestens eine funktionale funktionelle Gruppe ausgewählt aus O-C(=O)-Gruppe und O-C(=O)-O-Gruppe umfasst, und Urformen des Materials.

9. Verfahren nach dem vorhergehenden Anspruch, wobei das Urformen des Materials thermisches Urformen umfasst, wobei das Urformen bevorzugt mindestens ein Verfahren ist ausgewählt aus der Gruppe bestehend aus: Spritzgießen, Extrudieren, Kalandrieren, Rotationsformen, Spritzblasformen.

10. Verfahren zum Herstellen eines Fertigteils aus einem Material, wobei das Material ein Polyethylen-artiges Polymer aufweist, aufweist, wobei das Polyethylen-artige Polymer Methylengruppen (CH₂) und mindestens eine funktionale funktionelle Gruppe ausgewählt aus O-C(=O)-Gruppe und O-C(=O)-O-Gruppe umfasst, wobei das Fertigteil mittels eines additiven Fertigungsverfahrens hergestellt wird.

11. Verfahren nach dem vorhergehenden Anspruch, wobei das Material als Filament bereitgestellt wird und/oder wobei das additive Fertigungsverfahren 3-D-Drucken ist.

12. Verfahren nach einem der vorhergehenden Ansprüche, wobei das Polyethylen-artige Polymer, welches Methylengruppen (CH₂) und mindestens eine funktionale funktionelle Gruppe ausgewählt aus O-C(=O)-Gruppe und O-C(=O)-O-Gruppe umfasst, ein Polyethylen-artiges Polymer ist, wie in einem der Ansprüche 1 bis 5 zum Verfahren zur Herstellung von Monomeren beschrieben.

13. Polyethylen-artiges Polymer umfassend Methylengruppen (CH₂) und mindestens eine funktionelle Gruppe ausgewählt aus O-C(=O)-Gruppe und O-C(=O)-O-Gruppe, wobei das Polyethylen-artige Polymer ein zahlenmittleres Molekulargewicht (Mₙ) im Bereich von 20.000 bis 10.000.000 g/mol aufweist.

14. Polyethylen-artiges Polymer gemäß Anspruch 13, wobei 90 bis 99,5 Mol-% des Polyethylen-artigen Polymers aus Methyleneinheiten (CH₂) bestehen.

15. Polyethylen-artiges Polymer gemäß Anspruch 13 oder 14, aufweisend eine Wiederholungseinheit der allgemeinen Formel (I)
[-X-(CH₂)ₐ-] (I)
wobei
X ausgewählt ist aus C(=O)-O-Gruppe bzw. -O-C(=O)-Gruppe und -O-C(=O)-O-Gruppe;
a eine ganze Zahl aus dem Bereich von 8 bis 200 ist.
